# EUROPÄISCHE PATENTSCHRIFT

(11) **EP 0 770 587 B1**
(45) Veröffentlichungstag und Bekanntmachung des Hinweises auf die Patenterteilung: **22.03.2006**
(21) Anmeldenummer: 97101673.8
(22) Anmeldetag: 27.11.1992
(51) Int. Cl.: C07C 13/465, C07C 49/67, C07C 49/697, C07C 45/46, C07F 17/00

(54) **Verfahren zur Herstellung einer Indenverbindung und deren Verwendung zur Herstellung eines Metallocenkatalysator**
Process for the production of an indene compound and its use for the production of a metallocene catalyst
Méthode pour la préparation d'un composé d'indène et son utilisation pour la préparation d'un catalyseur métallocène

(30) Priorität: 30.11.1991 DE 4139596
(43) Veröffentlichungstag der Anmeldung: 02.05.1997
(62) Teilanmeldung aus: 92120288.3
(73) Patentinhaber: Basell Polyolefine GmbH, 50389 Wesseling (DE)
(72) Erfinder: Winter, Andreas, Dr., 61479 Glashütten (DE); Rohrmann, Jürgen, Dr., 65779 Kelkheim (DE); Dolle, Volker, Dr., 64625 Bensheim (DE); Küber, Frank, Dr., 61440 Oberursel (DE)

(56) Entgegenhaltungen:
- EP-A- 0 344 887
- DE-A- 2 039 426
- DE-A- 2 244 761
- JP-A- 56 084 731
- JP-A- 56 084 748
- DATABASE CROSSFIRE Beilstein Informationssysteme GmbH Frankfurt DE XP002032608 & BULLETIN DE LA SOCIETE CHIMIQUE DE FRANCE, 1970, Seiten 1466-1473,
- DATABASE CROSSFIRE Beilstein Informationssysteme GmbH Frankfurt DE XP002032609 & BULLETIN DE LA SOCIETE CHIMIQUE DE FRANCE, 1951, Seite 961, 964
- DATABASE CROSSFIRE Beilstein Informationssysteme GmbH Frankfurt DE XP002032610 & CHEMISCHE BERICHTE, Bd. 102, 1969, Seiten 2493-2501,
- DATABASE CROSSFIRE Beilstein Informationssysteme GmbH Frankfurt DE XP002032611 & JOURNAL OF ORGANIC CHEMISTRY, Bd. 40, Nr. 12, 1975, Seiten 1838-1840,
- DATABASE CROSSFIRE Beilstein Informationssysteme GmbH Frankfurt DE XP002032612 & HELVETICA CHIMICA ACTA, Bd. 32, 1949, Seite 2137, 2141
- DATABASE CROSSFIRE Beilstein Informationssysteme GmbH Frankfurt DE XP002032613 & BULLETIN DE LA SOCIETE CHIMIQUE DE FRANCE , 1969, Seiten 1981-1989,
- DATABASE CROSSFIRE Beilstein Informationssysteme GmbH Frankfurt DE XP002032614 & JOURNAL OF AMERICAN CHEMICAL SOCIETY, Bd. 92, Nr. 19, 1970, Seiten 5625-5640,
- DATABASE CROSSFIRE Beilstein Informationssysteme GmbH Frankfurt DE XP002032615 & JOURNAL OF ORGANIC CHEMISTRY, Bd. 51, Nr. 7, 1986, Seiten 964-970,
- DATABASE CROSSFIRE Beilstein Informationssysteme GmbH Frankfurt DE XP002032616 & BULLETIN DE LA SOCIETE CHIMIQUE DE FRANCE, 1965, Seiten 785-787,
- DATABASE CROSSFIRE Beilstein Informationssysteme GmbH Frankfurt DE XP002032617 & BULLETIN DE LA SOCIETE CHIMIQUE DE FRANCE , 1964, Seiten 3103-3112,
- DATABASE CROSSFIRE Beilstein Informationssysteme GmbH Frankfurt DE XP002032618 & BULLETIN DE LA SOCIETE CHIMIQUE DE FRANCE , 1964, Seiten 3103-3112,
- DATABASE CROSSFIRE Beilstein Informationssysteme GmbH Frankfurt DE XP002032619 & JOURNAL OF AMERICAN CHEMICAL SOCIETY, Bd. 62, 1940, Seite 2103, 2107
- DATABASE CROSSFIRE Beilstein Informationssysteme GmbH Frankfurt DE XP002032620 & HELVETICA CHIMICA ACTA, Bd. 32, 1949, Seite 2137, 2141
- DATABASE CROSSFIRE Beistein Informationssysteme GmbH Frankfurt DE XP002032621 & JOURNAL OF ORGANIC CHEMISTRY, Bd. 55, Nr. 7, 1990, Seiten 2132-2137,
- DATABASE CROSSFIRE Beilstein Informationssysteme GmbH Frankfurt DE XP002032622 & CHEMISCHE BERICHTE, Bd. 102, 1969, Seiten 2493-2501,
- DATABASE CROSSFIRE Beilstein Informationssysteme XP002032623 & INDIAN JOURNAL OF CHEMISTRY, Bd. 9, 1971, Seite 1183
- DATABASE CROSSFIRE Beilstein Informationssyisteme GmbH Frankfurt DE XP002032624 & INDIAN JOURNAL OF CHEMISTRY, Bd. 9, Nr. 1971, Seite 1183
- DATABASE CROSSFIRE Beilstein Informationssysteme GmbH Frankfurt DE XP002032625 & CHEMISCHE BERICHTE, Bd. 76, 1943, Seite 1157, 1158, 1160
- DATABASE CROSSFIRE Beilstein Informationssysteme GmbH Frankfurt DE XP002032626 & BULLETIN DE LA SOCIETE CHIMIQUE DE FRANCE, Nr. 6, 1987, Seiten 1079-1083,
- DATABASE CROSSFIRE Beilstein Informationssysteme GmbH Frankfurt DE XP002032627 & BULLETIN DE LA SOCIETE CHIMIQUE DE FRANCE, 1951, Seite 961, 964
- DATABASE CROSSFIRE Beilstein Informationssysteme GmbH Frankfurt DE XP002032628 & JOURNAL OF CHEMICAL SOCIETY, PERKIN TRANSACTIONS, 1979, Seiten 3034-3036,
- DATABASE CROSSFIRE Beilstein Informationssysteme GmbH Frankfurt DE XP002032629 & CHEMISCHE BERICHTE, Bd. 92, 1959, Seite 1662, 1666
- BULLETIN DE LA SOCIETE CHIMIQUE DE FRANCE. 1 PARTIE - CHIMIE ANALYTIQUE, MINERALE ET PHYSIQUE, Nr. 11, November 1973, Seiten 3092-3095, XP000647496 OLIVIER M ET AL: "ETUDE DE MONOMERES HALOGENES ET DE LEUR POLYMERISATION CATIONIQUE I. SYNTHESE DE DIVERS FLUORO-INDENES"
- JOURNAL OF THE AMERICAN CHEMICAL SOCIETY, Bd. 72, Nr. 7, 20.Juli 1950, Seiten 3286-3287, XP002032607 R.T. HART ET AL.: "Acylation-Alkylation Studies"

## Beschreibung

Die Erfindung bezieht sich auf ein Verfahren zur Herstellung einer Indenverbindung und deren Verwendung zur Herstellung eines Metallocens, welches zusammen mit einem Cokatalysator einen Katalysator für die Herstellung von Olefinpolymeren mit hoher Isotaktizität, enger Molmassenverteilung und hoher Molmasse bildet.
Weiterhin betrifft die Erfindung neue Indene und neue Indanone.

Polyolefine mit hoher Molmasse besitzen insbesondere Bedeutung für die Herstellung von Folien, Platten oder Großhohlkörpern wie z.B. Rohre oder Formteile.

Aus der Literatur ist die Herstellung von Polyolefinen mit löslichen Metallocenverbindungen in Kombination mit Aluminoxanen oder anderen Cokatalysatoren, die aufgrund ihrer Lewis-Acidität das neutrale Metallocen in ein Kation überführen und stabilisieren können, bekannt.

Beispielsweise wurde eine spezielle Voraktivierungsmethode des Metallocens mit einem Aluminoxan vorgeschlagen, welche zu einer beachtlichen Steigerung der Aktivität des Katalystorsystems und zu einer deutlichen Verbesserung der Kornmorphologie des Polymeren führt (vgl. DE 37 26 067). Die Voraktivierung erhöht zwar die Molmasse, jedoch ist keine wesentliche Steigerung erreichbar.

Eine weitere, aber noch nicht ausreichende, Steigerung der Molmasse konnte durch Verwendung speziell heteroatomverbrückter Metallocene bei hoher Metallocenaktivität realisiert werden (EP-A 0 336 128).

Weiterhin sind Katalysatoren auf Basis Ethylenbisindenylhafniumdichlorid und Ethylen-bis(4,5,6,7-tetrahydro-1-indenyl)hafniumdichlorid und Methylaluminoxan bekannt, mit denen durch Suspensionspolymerisation höhermolekulare Polypropylene hergestellt werden können (vgl. J.A. Ewen et al., J. Am.Chem.Soc. 109 (1987) 6544). Unter technisch relevanten Polymerisationsbedingungen ist jedoch die Kornmorphologie der derart erzeugten Polymeren nicht befriedigend und die Aktivität der eingesetzten Katalysatoren vergleichsweise gering. Verbunden mit den hohen Katalysatorkosten ist somit mit diesen Systemen eine kostengünstige Polymerisation nicht möglich.

Eine deutliche Steigerung der Molmasse konnte durch die Verwendung von Metallocenen erreicht werden, bei denen die durch eine Brücke fixierten aromatischen π-Liganden in 2-Stellung (DE-P 40 35 886.0) oder in 2- und 4-Stellung (DE-P 41 28 238.8) Substituenten tragen.

Unter dem Zwang großtechnisch kostengünstiger Produktion muß bei möglichst hohen Reaktionstemperaturen polymerisiert werden, da bei höheren Polymerisationstemperaturen die entstehende Polymerisationswärme mit weniger Kühlmedium abgeführt werden kann und daher mit einer deutlich geringeren Dimensionierung des Kühlwasserkreislaufes realisierbar ist.

Die letztgenannten Metallocene mit Substituenten in 2- bzw. 2- und 4-Stellung zur Brücke sind in dieser Hinsicht bei 70°C Polymerisationstemperatur bereits sehr leistungsfähig, trotzdem sind die erzielbaren Molmassen bei technisch relevanten Polymerisationstemperaturen (z.B. 70°C) für einige technische Anwendungen wie beispielsweise die Herstellung vcn Polymeren für Rohre und Großhohlkörper sowie spezielle Fasern noch zu gering.

Es bestand die Aufgabe, Indene bzw. ein Verfahren zur Herstellung von Indenen zu finden, die zur Herstellung eines Metallocens für ein Katalysatorsystem verwendet werden können, das Polymere mit guter Kornmorphologie und hoher Molmasse in großer Ausbeute erzeugt. Durch Verwendung von Wasserstoff als Molmassenregler kann dann die gesamte Molmassenpalette mit nur einem Metallocen abgedeckt werden.

Es wurde gefunden, daß unter Verwendung in bestimmter Weise substituierter Indene diese Aufgabe gelöst werden kann.

Die Erfindung betrifft somit die Verwendung eines Indens der Formel VII oder VIIa 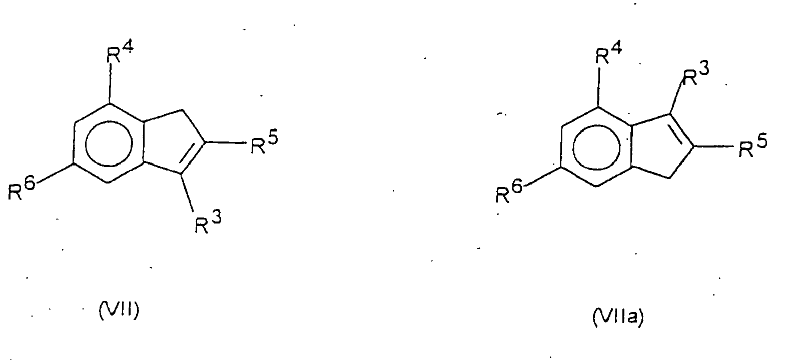 zur Herstellung eines Metallocens der Formel I 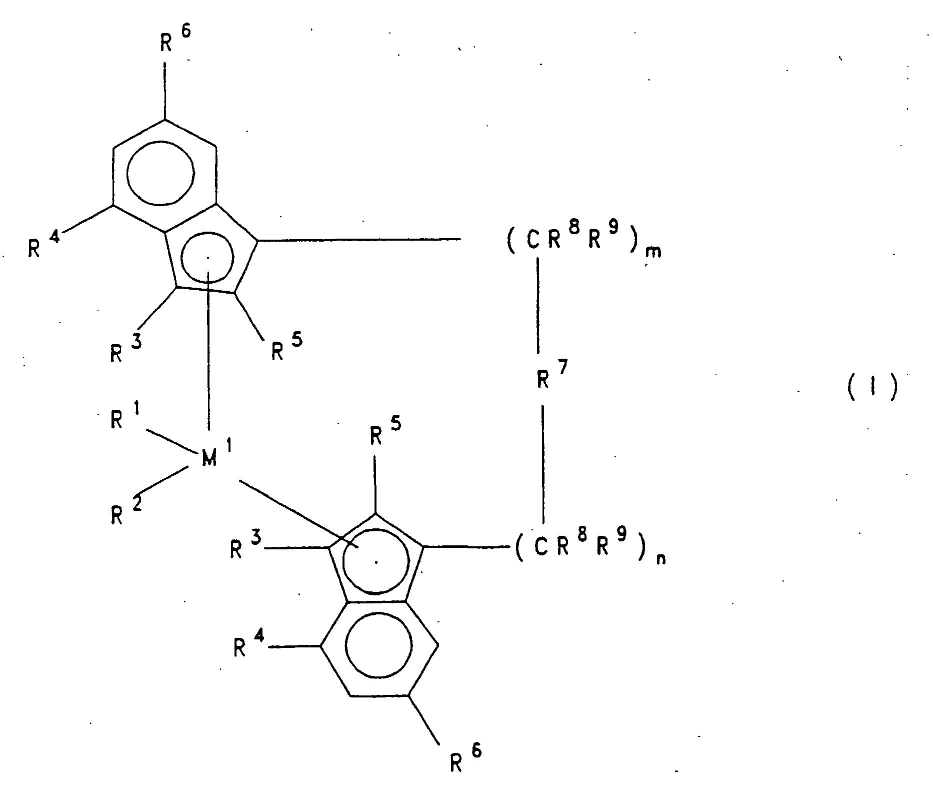 worin
- M¹: ein Metall der Gruppe IVb, Vb oder Vlb des Periodensystems ist,
- R¹ und R²: gleich oder verschieden sind und ein Wasserstoffatom, eine C₁-C₁₀-Alkylgruppe, eine C₁-C₁₀-Alkoxygruppe, eine C₆-C₁₀-Arylgruppe, eine C₆-C₁₀-Aryloxygruppe, eine C₂-C₁₀-Alkenylgruppe, eine C₇-C₄₀-Arylalkylgruppe, eine C₇-C₄₀-Alkylarylgruppe, eine C₈-C₄₀-Arylalkenylgruppe oder ein Halogenatom bedeuten,
die Reste R³ Wasserstoff bedeuten,
R⁴ bis R⁶ gleich oder verschieden sind und ein
Halogenatom, eine C₁-C₁₀-Alkylgruppe, die halogeniert sein kann, eine C₆-C₁₀-Arylgruppe, die halogeniert sein kann, einen -NR₂¹⁰, -SR¹⁰, -OSiR₃¹⁰, -SiR₃¹⁰ oder -PR₂¹⁰-Rest bedeuten, worin R¹⁰ ein Halogenatom, eine C₁-C₁₀₋Alkylgruppe oder eine C₆-C₁₀-Arylgruppe ist,
R⁷ 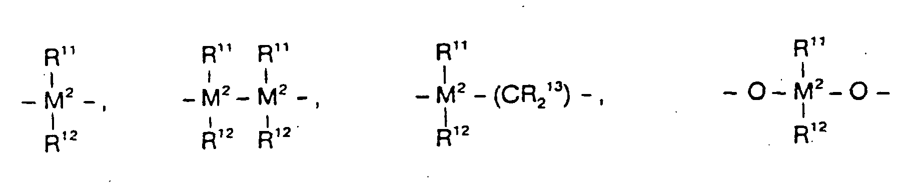 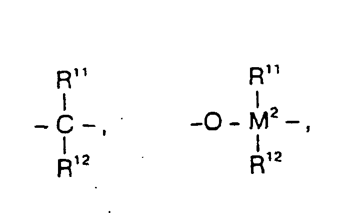 =BR¹¹, =AlR¹¹, -Ge-, -Sn-, -O-, -S-, =SO, =SO₂, =NR¹¹, =CO, =PR¹¹ oder =P(O)R¹¹ ist,
wobei
- R¹¹, R¹² und R¹³: gleich oder verschieden sind und ein Wasserstoffatom, ein Halogenatom, eine C₁-C₁₀-Alkylgruppe, C₁-C₁₀-Fluoralkylgruppe, eine C₆-C₁₀₋Arylgruppe, eine C₆-C₁₀-Fluorarylgruppe, eine C₁-C₁₀-Alkoxygruppe, eine C₂-C₁₀-Alkenylgruppe, eine C₇-C₄₀-Arylalkylgruppe, eine C₈-C₄₀₋Arylalkenylgruppe, eine C₇-C₄₀-Alkylarylgruppe bedeuten oder R¹¹ und R¹² oder R¹¹ und R¹³ jeweils mit den sie verbindenden Atomen einen Ring bilden,
- M²: Silizium, Germanium oder Zinn ist,
- R⁸ und R⁹: gleich oder verschieden sind und die für R¹¹ genannte Bedeutung haben und
- m und n: gleich oder verschieden sind und null, 1 oder 2 sind, wobei m plus n null, 1 oder 2 ist.

Alkyl steht für geradkettiges oder verzweigtes Alkyl. Halogen (halogeniert) bedeutet Fluor, Chlor, Brom oder Jod, bevorzugt Fluor oder Chlor.

Die Substituenten R⁴- R⁶ an den beiden Indenylresten können trotz gleicher Bezeichnung verschieden sein (vgl. Definition von R³).

Der für das Polymerisationsverfahren zu verwendende Katalysator besteht aus einem Cokatalysator und einem Metallocen der Formel I.

In Formel I ist M¹ ein Metall der Gruppe IVb, Vb oder VIb des Periodensystems, beispielsweise Titan, Zirkon, Hafnium, Vanadium, Niob, Tantal, Chrom, Molybdän, Wolfram, vorzugsweise Zirkon, Hafnium und Titan.

R¹ und R² sind gleich oder verschieden und bedeuten ein Wasserstoffatom, eine C₁-C₁₀-, vorzugsweise C₁-C₃-Alkylgruppe, eine C₁-C₁₀-, vorzugsweise C₁-C₃₋Alkoxygruppe, eine C₆-C₁₀-, vorzugsweise C₆-C₈-Arylgruppe, eine C₆-C₁₀-, vorzugsweise C₆-C₈-Aryloxygruppe, eine C₂-C₁₀-, vorzugsweise C₂-C₄₋Alkenylgruppe, eine C₇-C₄₀-, vorzugsweise C₇-C₁₀-Arylalkylgruppe, eine C₇-C₄₀-, vorzugsweise C₇-C₁₂-Alkylarylgruppe, eine C₈-C₄₀-, vorzugsweise C₈-C₁₂₋Arylalkenylgruppe oder ein Halogenatom, vorzugsweise Chlor.

Die Reste R³ sind gleich Wasserstoff.
Die Reste R⁴ bis R⁶ sind gleich oder verschieden und bedeuten ein Halogenatom, bevorzugt ein Fluor-, Chlor- oder Bromatom, eine C₁-C₁₀-, vorzugsweise C₁-C₄-Alkylgruppe, die halogeniert sein kann, eine C₆-C₁₀-, vorzugsweise C₆-C₉-Arylgruppe, die halogeniert sein kann, einen -NR₂¹⁰, -SR¹⁰, -OSiR₃¹⁰, -SiR₃¹⁰ oder -PR₂¹⁰-Rest, worin R¹⁰ ein Halogenatom, vorzugsweise Chloratom, oder eine C₁-C₁₀-, vorzugsweise C₁-C₃-Alkylgruppe oder C₆-C₁₀-, vorzugsweise C₆-C₈-Arylgruppe ist.

Bevorzugt sind R⁴ bis R⁶ (C₁-C₄)-Alkyl oder C₆-C₉-Aryl, die beide halogeniert sein können, wie Methyl, Ethyl, Propyl, Isopropyl, Butyl, Isobutyl, Trifluormethyl, Phenyl, Tolyl oder Mesityl, insbesondere Methyl, Isopropyl oder Phenyl.
R⁷ ist 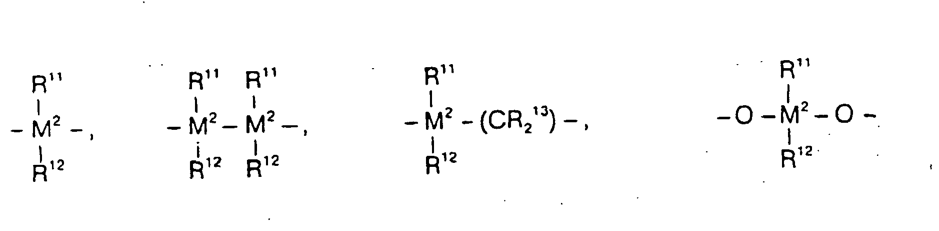 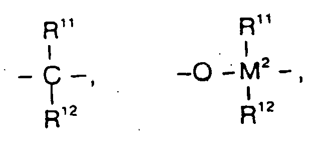 =BR¹¹, = AIR¹¹, -Ge-, -Sn-, -O-, -S-, =SO, = SO₂, =NR¹¹, = CO, =PR¹¹ oder =P(O)R¹¹, wobei R¹¹, R¹² und R¹³ gleich oder verschieden sind und ein Wasserstoffatom, ein Halogenatom, eine C₁-C₁₀-, vorzugsweise C₁-C₄-Alkylgruppe, insbesondere Methylgruppe, eine C₁-C₁₀-Fluoralkylgruppe, vorzugsweise CF₃₋Gruppe, eine C₆-C₁₀-, vorzugsweise C₆-C₈-Arylgruppe, eine C₆-C₁₀-Fluorarylgruppe, vorzugsweise Pentafluorphenylgruppe, eine C₁-C₁₀-, vorzugsweise C₁-C₄₋Alkoxygruppe, insbesondere Methoxygruppe, eine C₂-C₁₀-, vorzugsweise C₂-C₄₋Alkenylgruppe, eine C₇-C₄₀-, vorzugsweise C₇-C₁₀-Arylalkylgruppe, eine C₈-C₄₀-, vorzugsweise C₈-C₁₂-Arylalkenylgruppe oder eine C₇-C₄₀-, vorzugsweise C₇-C₁₂₋Alkylarylgruppe bedeuten, oder R¹¹ und A¹² oder R¹¹ und R¹³ bilden jeweils zusammen mit den sie verbindenden Atomen einen Ring.

M² ist Silizium, Germanium oder Zinn, bevorzugt Silizium und Germanium.

R⁷ ist vorzugsweise =CR¹¹R¹², =SiR¹¹R¹², =GeR¹¹R¹², -O-, -S-, =SO, =PR¹¹ oder =P(O)R¹¹.

R⁸ und R⁹ sind gleich oder verschieden und haben die für R¹¹ genannte Bedeutung.

m und n sind gleich oder verschieden und bedeuten null, 1 oder 2, bevorzugt null oder 1, wobei m plus n null, 1 oder 2, bevorzugt null oder 1 ist.

Somit sind die besonders bevorzugten Metallocene die Verbindungen der Formeln A, B und C 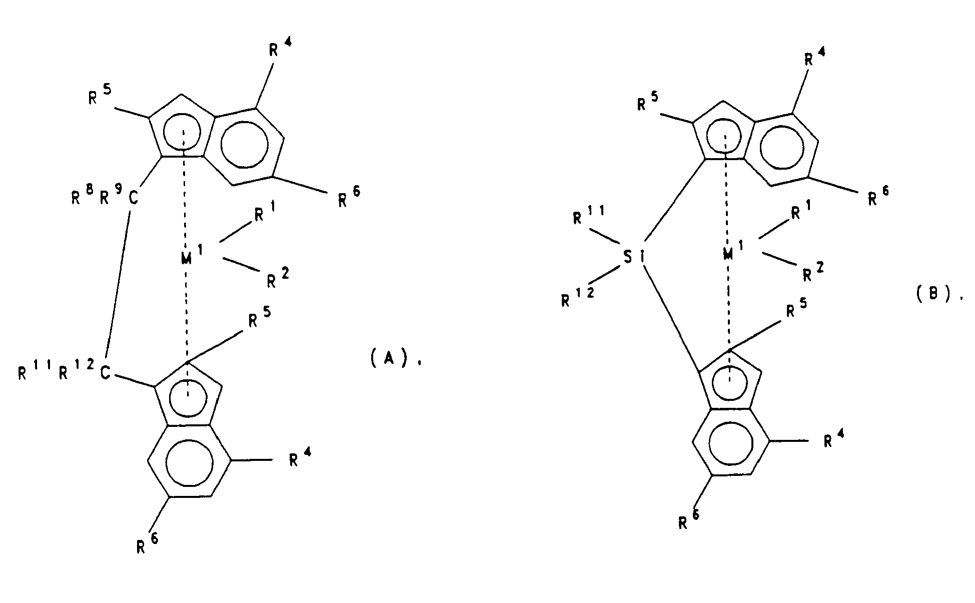 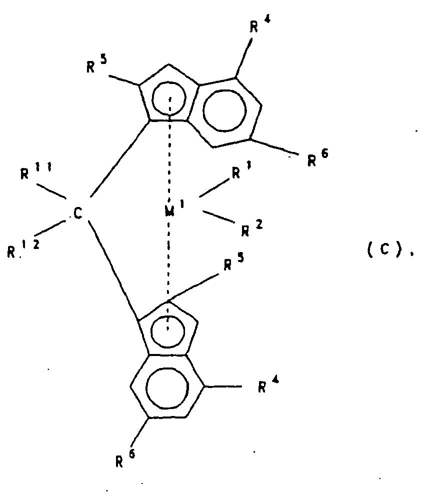 mit
M¹ = Zr, Hf; R¹, R² = Methyl, Chlor, R⁴ und R⁶ = Methyl, Isopropyl, Phenyl, Ethyl, Trifluormethyl; R⁵ = den für R⁴ und R⁶ genannten Bedeutungen, und R⁸, R⁹, R¹¹ und R¹² mit den obengenannten Bedeutungen, insbesondere die in den Ausführungsbeispielen aufgeführten Verbindungen I.

Das bedeutet, daß die Indenylreste der Verbindungen I in 2,4,6-Stellung substituiert sind.

Die chiralen Metallocene werden bevorzugt als Racemat eingesetzt. Verwendet werden kann aber auch die reine R- oder S-Form. Mit diesen reinen stereoisomeren Formen ist optisch aktives Polymeres herstellbar. Abgetrennt werden sollte jedoch die meso-Form der Metallocene, da das polymerisationsaktive Zentrum (das Metallatom) in diesen Verbindungen wegen Spiegelsymmetrie am Zentralmetall nicht mehr chiral ist und daher kein hochisotaktisches Polymeres erzeugen kann: Wird die meso-Form nicht abgetrennt, entsteht neben isotaktischen Polymeren auch ataktisches Polymer. Für bestimmte Anwendungen - weiche Formkörper beispielsweise - kann dies durchaus wünschenswert sein.

Die Trennung der Stereoisomeren ist im Prinzip bekannt.

Die vorstehend beschriebenen Metallocene können nach folgendem Reaktionsschema hergestellt werden: 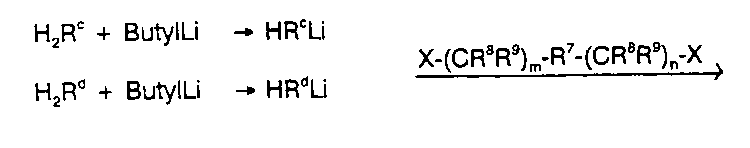 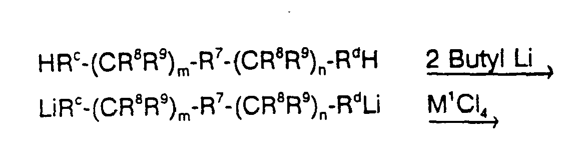 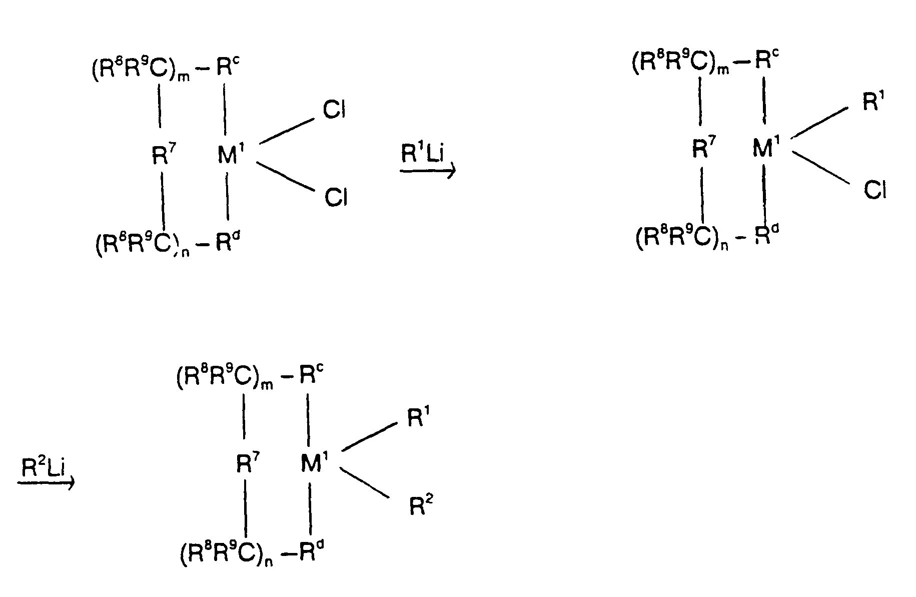 X = Cl, Br, l, 0-Tosyl;

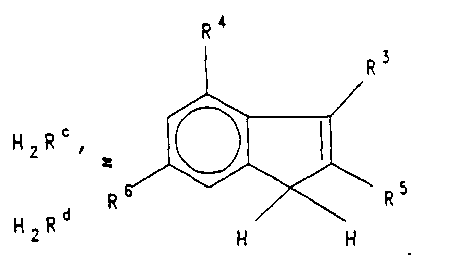

Die Herstellungsverfahren sind ausgehend von H₂R^{c}/H₂R^{d} literaturbekannt; vgl. Journal of Organometallic Chem. 288 (1985) 63-67, EP-A 320 762 und die Ausführungsbeispiele.

Weiterhin betrifft die Erfindung ein Verfahren zur Herstellung eines Indens der Formel VII oder VIIa 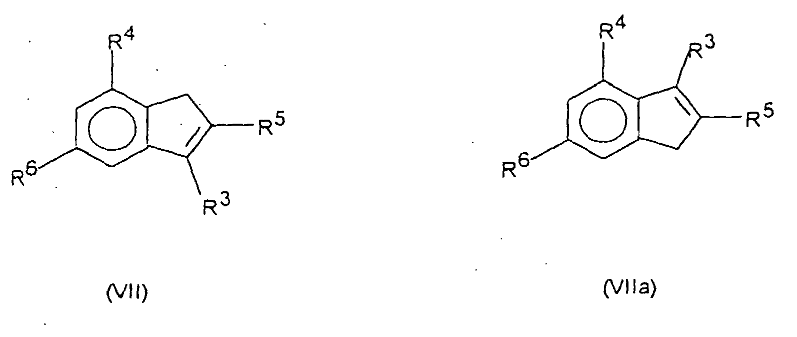 wobei
a) eine Verbindung IV 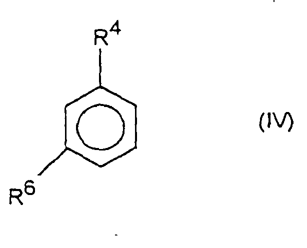 mit einer Verbindung V 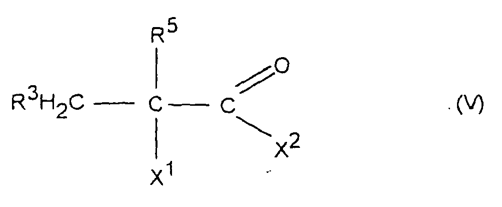 oder deren Anhydrid in Gegenwart eines Friedel-Crafts-Katalysators umgesetzt wird zu einem Indanon VI bzw. VIa 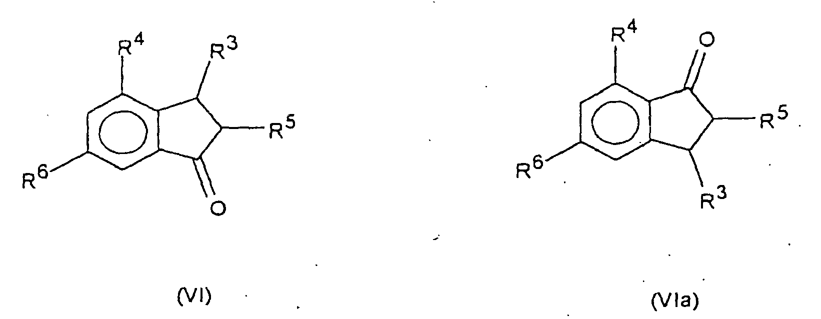 wobei in den Formel IV, V, VI, VIa, VII und VIIa die Reste R³ bis R⁶ wie in Formel I definiert sind und X¹ und X² für eine nucleophile Abgangsgruppe stehen, und
b) das Indanon anschließend mit einem Reduktionsmittel zu dem entsprechenden Indanol reduziert wird und dann dehydratisiert wird.

Dabei stehen
X¹ und X² für eine nucleophile Abgangsgruppe wie z.B. Halogen, Hydroxygruppe oder eine Tosylgruppe; insbesondere für Brom oder Chlor.

Die Indanone können in Abhängigkeit des Substitutionsmusters am Aromaten in Form zweier Konstitutionsisomere der Formel VI bzw. VIa anfallen. Diese können in reiner Form oder als Gemisch nach literaturbekannten Methoden mit Reduktionsmitteln wie NaBH₄ oder LiAlH₄ zu den entsprechenden Indanolen reduziert und anschließend mit Säuren wie Schwefelsäure, Oxalsäure, p-Toluolsulfonsäure oder auch durch Behandlung mit wasserentziehenden Substanzen wie Magnesiumsulfat, Natriumsulfat, Aluminiumoxid, Kieselgel oder Molekularsiebe zu Indenen der Formel VII bzw. VIIa (H₂R^{c}/H₂R^{d}) dehydratisiert werden (Bull. Soc. Chim. Fr. 11 (1973) 3092; Organomet. 9 (1990) 3098 und die Ausführungsbeispiele).

Geeignete Friedel-Crafts-Katalysatoren sind z.B. AlCl₃, AlBr₃, FeCl₃, SbCl₅, SnCl₄, BF₃, TiCl₄, ZnCl₂, HF, H₂SO₄, Polyphosphorsäure, H₃PO₄ oder eine AlCl₃/NaCl-Schmelze; insbesondere AlCl₃.

Die Ausgangsverbindungen der Formeln IV und V sind bekannt und im Handel erhältlich, oder sie lassen sich nach literaturbekannten Verfahren herstellen.

Die Umsetzung wird in einem inerten Lösemittel durchgeführt. Bevorzugt wird Methylenchlorid oder CS₂ eingesetzt. Sind die Ausgangskomponenten flüssig, kann auch auf ein Lösemittel verzichtet werden.

Die Molverhältnisse der Ausgangsverbindungen einschließlich des Friedel-Crafts-Katalysators können innerhalb weiter Grenzen schwanken. Bevorzugt ist das Molverhältnis von Verbindung I:II:Katalysator = 1:0,5-1,5:1-5; insbesondere 1:1:2,5-3.

Die Reaktionstemperatur beträgt bevorzugt 0°C bis 130°C, insbesondere 25°C bis 80°C.

Die Reaktionszeiten schwanken in der Regel zwischen 30 min und 100 h, vorzugsweise zwischen 2 h und 30 h.

Bevorzugt wird eine Mischung der Verbindungen IV und V vorgelegt und der Friedel-Crafts-Katalysator zudosiert. Auch die umgekehrte Zugabefolge ist möglich.

Die Indanone der Formel VI bzw. VIa können durch Destillation, Säulenchromatographie oder Kristallisation gereinigt werden.

Die substituierten Indene können als Doppelbindungsisomere anfallen (VII/VIIa). Diese können von Nebenprodukten durch Destillation, Säulenchromatographie oder Kristallisation gereinigt werden.

Ein weiterer Gegenstand der Erfindung sind Indene der Formel VII oder VIIa 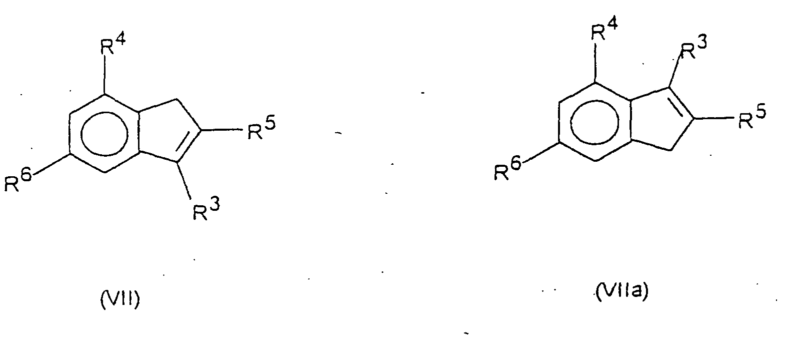 worin
R³ ein Wasserstoffatom ist,
R⁴ bis R⁶ gleich oder verschieden sind und ein Halogenatom, eine C₁-C₁₀₋Alkylgruppe, die halogeniert sein kann, eine C₆-C₁₀-Arylgruppe, die halogeniert sein kann, einen -NR¹⁰₂, -SR¹⁰, -OSiR¹⁰₃, -SiR¹⁰₃ oder -PR¹⁰₂-Rest bedeuten, worin R¹⁰ ein Halogenatom, eine C₁-C₁₀-Alkylgruppe oder ein C₆-C₁₀₋Arylgruppe ist, wobei 2,4,6-Trichlorinden ausgeschlossen ist.

Ebenfalls Gegenstand der Erfindung sind Indanone der Formel Via 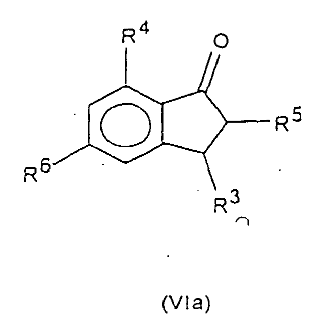 worin
- R³: ein Wasserstoffatom, ein Halogenatom, eine C₁-C₁₀-Alkylgruppe, die halogeniert sein kann, eine C₆-C₁₀-Arylgruppe, die halogeniert sein kann, einen -NR₂¹⁰, -SR¹⁰, -OSiR₃¹⁰, -SiR₃¹⁰ oder -PR₂¹⁰-Rest bedeutet, worin R¹⁰ ein Halogenatom, eine C₁-C₁₀-Alkylgruppe oder eine C₆-C₁₀-Arylgruppe ist,
- R⁴ bis R⁶: gleich oder verschieden sind und die für R³ genannte Bedeutung haben, mit der Maßgabe, daß R⁴ und R⁶ kein Wasserstoffatom sind
bevorzugt Indanone der Formel VIa, worin R³ ein Wasserstoffatom ist und R⁵ kein Wasserstoffatom ist, insbesondere 2-Methyl-4,6-diisopropyl-1-indanon, sowie die Verwendung eines Indanons der Formel VIa zur Herstellung eines Indens wie oben beschrieben.

Ausgehend von den Indenen der Formeln VII und VIIa, die als Isomerengemisch eingesetzt werden können, verläuft die Herstellung der Metallocene I nach literaturbekannten Verfahren (vgl. AU-A-31478/89, J. Organomet. Chem. 342 (1988) 21, EP-A 284 707 und die Ausführungsbeispiele) entsprechend dem angegebenen Reaktionsschema.

Als Cokatalysator wird bevorzugt ein Aluminoxan der Formel (II) 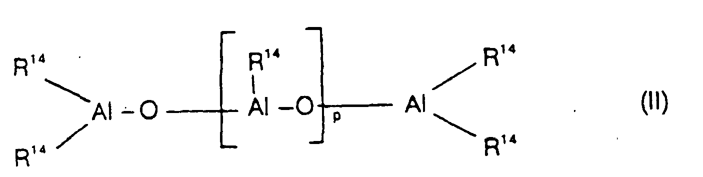 für den linearen Typ und/oder der Formel (III) 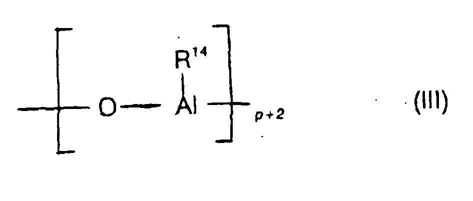 für den cyclischen Typ verwendet, wobei in den Formeln (II) und (III) die Reste R¹⁴ gleich oder verschieden sein können und eine C₁-C₆-Alkylgruppe, eine C₆-C₁₈₋Arylgruppe, Benzyl oder Wasserstoff bedeuten, und p eine ganze Zahl von 2 bis 50, bevorzugt 10 bis 35 bedeutet.

Bevorzugt sind die Reste R¹⁴ gleich und bedeuten Methyl, Isobutyl, Phenyl oder Benzyl, besonders bevorzugt Methyl.

Sind die Reste R¹⁴ unterschiedlich, so sind sie bevorzugt Methyl und Wasserstoff oder alternativ Methyl und Isobutyl, wobei Wasserstoff bzw. Isobutyl bevorzugt zu 0,01 - 40 % (Zahl der Reste R") enthalten sind.

Das Aluminoxan kann auf verschiedene Arten nach bekannten Verfahren hergestellt werden. Eine der Methoden ist beispielsweise, daß eine Aluminiumkohlenwasserstoffverbindung und/oder eine Hydridoaluminiumkohlenwasserstoffverbindung mit Wasser (gasförmig, fest, flüssig oder gebunden - beispielsweise als Kristallwasser) in einem inerten Lösungsmittel (wie z.B. Toluol) umgesetzt wird. Zur Herstellung eines Aluminoxans mit veschiedenen Alkylgruppen R¹⁴ werden entsprechend der gewünschten Zusammensetzung zwei verschiedene Aluminiumtrialkyle (AlR₃ + AlR'₃) mit Wasser umgesetzt (vgl. S. Pasynkiewicz, Polyhedron 9 (1990) 429 und EP-A 302 424).

Die genaue Struktur der Aluminoxane II und III ist nicht bekannt.

Unabhängig von der Art der Herstellung ist allen Aluminoxanlösungen ein wechselnder Gehalt an nicht umgesetzter Aluminiumausgangsverbindung, die in freier Form oder als Addukt vorliegt, gemeinsam.

Es ist möglich, das Metallocen vor dem Einsatz in der Polymerisationsreaktion mit einem Aluminoxan der Formel (II) und/oder (III) vorzuaktivieren. Dadurch wird die Polymerisationsaktivität deutlich erhöht und die Kornmorphologie verbessert.

Die Voraktivierung der Übergangsmetallverbindung wird in Lösung vorgenommen. Bevorzugt wird dabei das Metallocen in einer Lösung des Aluminoxans in einem inerten Kohlenwasserstoff aufgelöst. Als inerter Kohlenwasserstoff eignet sich ein aliphatischer oder aromatischer Kohlenwasserstoff. Bevorzugt wird Toluol verwendet.

Die Konzentration des Aluminoxans in der Lösung liegt im Bereich von ca. 1 Gew.-% bis zur Sättigungsgrenze, vorzugsweise von 5 bis 30 Gew.-%, jeweils bezogen auf die Gesamtlösung. Das Metallocen kann in der gleichen Konzentration eingesetzt werden, vorzugsweise wird es jedoch in einer Menge von 10⁻⁴- 1 mol pro mol Aluminoxan eingesetzt. Die Voraktivierungszeit beträgt 5 Minuten bis 60 Stunden, vorzugsweise 5 bis 60 Minuten. Man arbeitet bei einer Temperatur von -78°C bis 100°C, vorzugsweise 0 bis 70°C.

Das Metallocen kann auch vorpolymerisiert oder auf einen Träger aufgebracht werden. Zur Vorpolymerisation wird bevorzugt das (oder eines der) in der Polymerisation eingesetzte(n) Olefin(e) verwendet.

Geeignete Träger sind beispielsweise Silikagele, Aluminiumoxide, festes Aluminoxan oder andere anorganische Trägermaterialien. Ein geeignetes Trägermaterial ist auch ein Polyolefinpulver in feinverteilter Form.

An Stelle oder neben eines Aluminoxans können Verbindungen der Formeln RₓNH₄₋ₓBR'₄, RₓPH₄₋ₓBR'₄, R₃CBR'₄ oder BR'₃ als geeignete Cokatalysatoren verwendet werden. In diesen Formeln bedeutet x eine Zahl von 1 bis 4, bevorzugt 3, die Reste R sind gleich oder verschieden, bevorzugt gleich, und bedeuten C₁-C₁₀-Alkyl, C₆-C₁₈-Aryl oder 2 Reste R bilden zusammen mit dem sie verbindenden Atom einen Ring, und die Reste R' sind gleich oder verschieden, bevorzugt gleich, und stehen für C₆-C₁₈-Aryl, das durch Alkyl, Haloalkyl oder Fluor substituiert sein kann.

Insbesondere steht R für Ethyl, Propyl, Butyl oder Phenyl und R' für Phenyl, Pentafluorphenyl, 3,5-Bistrifluormethylphenyl, Mesityl, Xylyl oder Tolyl (vgl. EP-A 277 003, EP-A 277 004 und EP-A 426 638).

Bei Verwendung der obengenannten Cokatalysatoren besteht der eigentliche (aktive) Polymerisationskatalysator aus dem Reaktionsprodukt von Metallocen und einer der genannten Verbindungen. Daher wird zunächst dieses Reaktionsprodukt bevorzugt außerhalb des Polymerisationsreaktors in einem separaten Schritt unter Verwendung eines geeigneten Lösungsmittels hergestellt.

Prinzipiell ist als Cokatalysator erfindungsgemäß jede Verbindung geeignet, die aufgrund ihrer Lewis-Acidität das neutrale Metallocen in ein Kation überführen und dieses stabilisieren kann ("labile Koordination"). Darüberhinaus soll der Cokatalysator bzw. das aus ihm gebildete Anion keine weiteren Reaktionen mit dem gebildeten Metallocenkation eingehen (vgl. EP-A 427 697).

Zur Entfernung von im Olefin vorhandener Katalystorgifte ist eine Reinigung mit einem Aluminiumalkyl, beispielsweise AlMe₃ oder AlEt₃ vorteilhaft. Diese Reinigung kann sowohl im Polymerisationssystem selbst erfolgen, oder das Olefin wird vor der Zugabe in das Polymerisationssystem mit der Al-Verbindung in Kontakt gebracht und anschließend wieder abgetrennt.

Die Polymerisation oder Copolymerisation wird in bekannter Weise in Lösung, in Suspension oder in der Gasphase, kontinuierlich oder diskontinuierlich, ein- oder mehrstufig bei einer Temperatur von -60 bis 200°C, vorzugsweise 30 bis 80°C, besonders bevorzugt 50 bis 80°C, durchgeführt. Polymerisiert oder copolymerisiert werden Olefine der Formel R^{a}-CH=CH-R^{b}. In dieser Formel sind R^{a} und R^{b} gleich oder verschieden und bedeuten ein Wasserstoffatom oder einen Alkylrest mit 1 bis 14 C-Atomen. R^{a} und R^{b} können jedoch auch mit den sie verbindenden C-Atomen einen Ring bilden. Beispiele für solche Olefine sind Ethylen, Propylen, 1-Buten, 1-Hexen, 4-Methyl-1-penten, 1-Octen, Norbornen oder Norbonadien. Insbesondere werden Propylen und Ethylen polymerisiert.

Als Molmassenregler und/oder zur Aktivitätserhöhung wird, falls erforderlich, Wasserstoff zugegeben. Der Gesamtdruck im Polymerisationssystem beträgt 0,5 bis 100 bar. Bevorzugt ist die Polymerisation in dem technisch besonders interessanten Druckbereich von 5 bis 64 bar.

Dabei wird das Metallocen in einer Konzentration, bezogen auf das Übergangsmetall, von 10⁻³ bis 10⁻⁸, vorzugsweise 10⁻⁴ bis 10⁻⁷ mol Übergangsmetall pro dm³ Lösemittel bzw. pro dm³ Reaktorvolumen angewendet. Das Aluminoxan wird in einer Konzentration von 10⁻⁵ bis 10⁻¹ mol, vorzugsweise 10⁻⁴ bis 10⁻² mol pro dm³ Lösemittel bzw. pro dm³ Reaktorvolumen verwendet. Die anderen genannten Cokatalysatoren werden in etwa äquimolaren Mengen zum Metallocen verwendet. Prinzipiell sind aber auch höhere Konzentrationen möglich.

Wenn die Polymerisation als Suspensions- oder Lösungspolymerisation durchgeführt wird, wird ein für das Ziegler- Niederdruckverfahren gebräuchliches inertes Lösemittel verwendet. Beispielsweise arbeitet man in einem aliphatischen oder cycloaliphatischen Kohlenwasserstoff; als solcher sei beispielsweise Propan, Butan, Pentan, Hexan, Heptan, Isooctan, Cyclohexan, Methylcyclohexan, genannt.

Weiterhin kann eine Benzin- bzw. hydrierte Dieselölfraktion benutzt werden. Brauchbar ist auch Toluol. Bevorzugt wird im flüssigen Monomeren polymerisiert.

Werden inerte Lösemittel verwendet, werden die Monomeren gasförmig oder flüssig zudosiert.

Die Dauer der Polymerisation ist beliebig, da das erfindungsgemäß zu verwendende Katalysatorsystem einen nur geringen zeitabhängigen Abfall der Polymerisationsaktivfiät zeigt.

Das Polymerisationsverfahren zeichnet sich dadurch aus, daß die beschriebenen Metallocene im technisch besonders interessanten Temperaturbereich zwischen 50 und 80°C Polymere mit hoher Molmasse, hoher Stereospezifität und guter Kornmorphologie erzeugen.

Die Zirkonocene stoßen in einen Molmassenbereich vor, oder übertreffen diesen sogar, der beim bisherigen Stand der Technik den Hafnocenen vorbehalten war. Diese hatten jedoch den Nachteil nur geringer Polymerisationsaktivität und sehr hoher Katalysatorkosten und die damit hergestellten Polymeren wiesen eine schlechte Pulvermorphologie auf.

Die nachfolgenden Beispiele sollen die Erfindung näher erläutern.

Es bedeuten:
- VZ =: Viskositätszahl in cm³/g 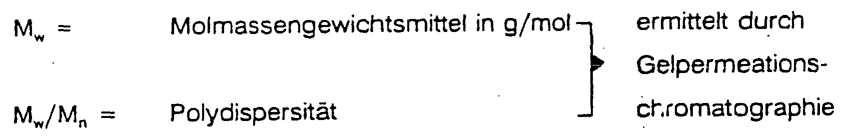
- Schmp. =: Schmelzpunkt ermittelt mit DSC (20°C/min Aufheiz-/Abkühlgeschwindigkeit)
- II =: Isotaktischer Index (II = mm+1/2 mr) ermittelt durch ¹³C-NMR-Spektroskopie
- MFI/(230/5): Schmelzindex, gemessen nach DIN 53735; in dg/min
- SD =: Polymerschüttdichte in g/dm³

Synthese der in den Beispielen verwendeten Metallocene:

### Beispiel A

### 2,5,7-Trimethyl-1-indanon (1)

Eine Lösung von 34,4 g (324 mmol) m-Xylol (99 %) und 74 g (324 mmol) 2-Bromisobuttersäurebromid (98 %) in 600 ml Methylenchlorid p. a. wurde bei Raumtemperatur unter kräftigem Rühren über einen Feststoffdosiertrichter langsam mit 107 g (810 mmol) AlCl₃ versetzt, wobei eine heftige Gasentwicklung einsetzte. Die Mischung wurde 15 h bei Raumtemperatur gerührt, auf Eiswasser gegossen, das mit 25 ml konz. HCl angesäuert wurde und mehrmals ausgeethert. Die vereinigten organischen Phasen wurden zunächst mit einer gesättigten NaHCO₃₋Lösung und dann mit einer gesättigten NaCl-Lösung gewaschen und mit Magnesiumsulfat getrocknet. Das nach Abziehen des Lösemittels unter vermindertem Druck zurückgebliebene Öl wurde über eine kurze Destillationsbrücke destilliert. Bei 81-90°C/0,1-0,2 mbar gingen 52,4 g des Indanons 1 in Form eines farblosen Öls über, das bei Raumtemperatur kristallisierte. Die Ausbeute betrug 93%.
¹H-NMR-Spektrum (100 MHz, CDCl₃): 7,05 (1,s), 6,87 (1,s), 3,25 (1,q), 2,43-2,80 (2,m), 2,57 (3,s), 2,35 (3,s), 1,25 (3,d).
Massenspektrum: 174 M⁺, korrektes Zerfallsmuster.

### Beispiel B

### 2,4,6-Trimethylinden (2)

20,4 g (117 mmol) 2,5,7-Trimethyl-1-indanon (1) wurden in 300 ml einer Mischung aus Tetrahydrofuran/Methanol (2:1) gelöst und bei Raumtemperatur mit 6,6 g (175 mmol) NaBH₄ versetzt. Die Mischung wurde noch 1 h gerührt, mit 50 ml halbkonz. HCl versetzt und ausgeethert. Die vereinigten organischen Phasen wurden über Natriumsulfat getrocknet und vom Lösemittel befreit. Der Rückstand wurde in eine Destillationsapparatur überführt und mit 13 g Magnesiumsulfat versetzt. Es wurde ein Vakuum von ca. 10 mbar angelgt und hochgeheizt, bis das Produkt überdestillierte (130-150°C). Die Destillation ergab 17,7 g des Indens 2 als farbloses Öl. Die Ausbeute betrug 96 %.
Massenspektrum: 158 M⁺, korrektes Zerfallsmuster.

### Beispiel C

### 2-Methyl-5,7-diisopropyl-1-indanon (3) bzw. 2-Methyl-4,6-diisopropyl-1-indanon (3a)

Eine Lösung von 84,8 g (523 mmol) 1,3-Diisopropylbenzol und 120 g (523 mmol) 2-Bromisobuttersäurebromid (98 %) in 600 ml Methylenchlorid p. a. wurde bei Raumtemperatur über einen Feststoffdosiertrichter langsam mit 174 g (1300 mmol) AlCl₃ versetzt. Die Mischung wurde noch 20 h zum Rückfluß erhitzt und dann analog Beispiel A aufgearbeitet. Das Rohprodukt wurde an 3 kg Kieselgel 60 chromatographiert. Die Indanone 3 und 3a konnten mit einem Laufmittelgemisch aus Hexan/15 % Essigester separat eluiert werden. Mit dem gleichen Laufmittel folgte in einer weiteren Zone als Nebenprodukt die Verbindung 2-Methyl-5-isopropyl-1-indanon. Eine Trennung der beiden Isomere ist für die weiteren Umsetzungen jedoch nicht erforderlich. Die Gesamtausbeute betrug 93 g (78 %).
¹H-NMR-Spektrum (360 MHz, CDCl₃): Isomerenmischung (3:2) 7,49 (d), 7,36 (d), 7,13 (s), 7,10 (s), 4,15 (sept.), 3,25-3,40 (m), 3,10 (sept.), 2,90-3,00 (m), 2,60-2,73 (m), 1,22-1,30 (m).
Massenspektrum: 230 M⁺, korrektes Zerfallsmuster.

### Beispiel D

### 2-Methyl-4,6-diisopropylinden (4) bzw. 2-Methyl-5,7-diisopropylinden (4a), Variante I

Eine Lösung von 78,5 g (341 mmol) der Isomerenmischung 3/3a in 700 ml eines Lösemittelgemisches aus Tetrahydrofuran/Methanol p.a. (2:1) wurde bei Raumtemperatur mit 19,3 g (511 mmol) NaBH₄ versetzt. Nach 2 h Rühren bei Raumtemperatur wurden 120-130 ml halbkonz. HCl zugesetzt und ausgeethert. Die vereinigten organischen Phasen wurden mit Na₂SO₄ getrocknet. Der nach Abziehen des Lösemittels verbliebene Rückstand wurde in 500 ml Methylenchlorid aufgenommen und mit 6,5 g (34 mmol) p-Toluolsulfonsäure 15 min zum Rückfluß erhitzt. Der nach Abziehen des Lösemittels verbliebene Rückstand wurde an 1,5 kg Kieselgel 60 chromatographiert. Mit einem Laufmittelgemisch aus Hexan/Diisopropylether 20:1 ließen sich 56 g der Isomerenmischung 4/4a in Form eines farblosen Öls isolieren. Die Gesamtausbeute betrug 86 %.
¹H-NMR-Spektrum (100 MHz, CDCl₃): Doppelbindungsisomere (1:1) 7,1 (m), 6,95 (m), 6,60 (m), 6,43 (m), 3,25 (br), 2,75-3,20 (m), 2,12 (d), 1,28 (d), 1,25 (d).
Massenspektrum: 214 M⁺, korrektes Zerfallsmuster.

### Beispiel E

### 2-Methyl-4,6-diisopropylinden (4) bzw. 2-Methyl-5,7-diisopropylinden (4a), Variante II

Eine Lösung von 78,5 g (341 mmol) der Isomerenmischung 3/3a in 700 ml eines Lösemittelgemisches aus Tetrahydrofuran/Methanol p.a. (2:1) wurde bei Raumtemperatur mit 19,3 g (511 mmol) NaBH₄ versetzt. Nach 2 h Rühren bei Raumtemperatur wurden 120-130 ml halbkonz. HCl zugesetzt und ausgeethert. Die vereinigten organischen Phasen wurden mit Na₂SO₄ getrocknet. Der nach Abziehen des Lösemittels verbliebene Rückstand wurde in eine Destillationsapparatur überführt und mit 50 g Magnesiumsulfat versetzt. Nach Anlegen eines Vakuums von ca. 1 mbar wurde hochgeheizt, bis das Produkt überging (ca. 130°C). Man erhielt 65 g der Isomerenmischung 4/4a als farbloses Öl. Die Ausbeute betrug 90%.

### Beispiel F

### Dimethylbis(2-Methyl-4,6-diisopropylindenyl)silan (5)

Eine Lösung von 4,9 g (22,8 mmol) der Isomerenmischung 4/4a in 25 ml THF wurde bei 0°C unter Ar-Schutz mit 9,2 ml (22,8 mmol) einer 2,5 M Butyllithiumlösung in Hexan versetzt und noch 1 h zum Rückfluß erhitzt. Die rote Lösung wurde anschließend bei Raumtemperatur zu einer Lösung von 1,5 g (11,4 ml) Dimethyldichlorsilan in 10 ml THF getropft und 8 h unter Rückfluß erhitzt. Der Ansatz wurde auf Eiswasser gegossen und ausgeethert. Die über Magnesiumsulfat getrocknete Etherphase wurde unter vermindertem Druck eingedampft. Das zurückgebliebene gelbliche Öl wurde anschließend an 500 g Kieselgel 60 chromatographiert. Mit einem Laufmittelgemisch aus Hexan/5 % Methylenchlorid konnten zunächst 1,4 g der Indenmischung 4/4a eluiert werden. Mit Hexan/8 % Methylenchlorid folgte das Ligandsystem 11. Das nach Abziehen des Laufmittels verbliebene viskose Öl konnte durch Verrühren mit Methanol im Eisbad kristallisiert werden. Es wurden 3,1 g eines gelblichen Feststoffs erhalten. Die Ausbeute betrug 56 % bzw. 84 % bezüglich umgesetztem Inden.
¹H-NMR-Spektrum (100 MHz, CDCl₃): Doppelbindungsisomere (3:1) 6,82-7,32 (m), 6,70 (m), 6,62 (m), 6,52 (m), 3,75 (s,br), 3,65 (s,br), 3,35 (s), 2,70-3,30 (m), 2,05-2,25 (m), 1,10-1,45 (m), 0,10-0,22 (m), -0,15 bis -0,32 (m).
Massenspektrum: 484 M⁺, korrekter Zerfall.

### Beispiel G

### Dimethylsilandiyibis(2-Methyl-4,6-diisopropylindenyl)zirkondichlorid (6)

Eine Lösung von 3,1 g (6,5 mmol) des Ligandsystems 5 in 25 ml Diethylether wurde bei Raumtemperatur unter Ar-Schutz mit 6,3 ml (16,2 mmol) einer 2,5 M Butyllithiumlösung in Hexan versetzt und über Nacht gerührt. Nach Zusatz von 10 ml Hexan wurde die beigefarbene Suspension filtriert und der Rückstand mit 20 ml Hexan gewaschen. Das Dilithiosalz wurde lange im Ölpumpenvakuum getrocknet und dann bei -78°C zu einer Suspension von 1,6 g (6,8 mmol) ZrCl₄ in 30 ml Methylenchlorid gegeben. Die Mischung wurde innerhalb 1 h auf Raumtemperatur erwärmt und noch 30 min bei dieser Temperatur gerührt. Nach dem Abziehen des Lösemittels wurde der orangebraune Rückstand mit 50 ml Hexan extrahiert. Nach Abziehen des Lösemittels erhielt man 2,6 g (60 %) des Komplexes 12 in Form eines gelben Pulvers. Das Verhältnis des Racemats zur meso-Form war 3:1. Durch Umkristallisation aus Hexan konnten 1,3 g (30 %) des Komplexes 12 als reines Racemat gewonnen werden (gelbes Kristallpulver).
¹H-NMR-Spektrum (100 MHz, CDCl₃): 7,27 (2,s,Arom.-H), 7,05 (2,s,Arom.-H), 6,80 (2,s,ß-Ind-H), 2,6-3,2 (4,m,i-Pr-CH), 2,22 (6,s,Ind-CH₃). 1,15-1,40 (30,m,i-Pr-CH₃, Si-CH₃). Massenspektrum: 642 M⁺ (bzgl.⁹⁰Zr), korrektes Isotopenmuster, korrekter Zerfall.

### Beispiel H

### Dimethylbis(2,4,6-trimethylindenyl)silan (7)

Eine Lösung von 10,1 g (64 mmol) des Indens 2 in 50 ml THF wurde bei Raumtemperatur unter Ar-Schutz mit 25,5 ml (63,7 mmol) einer 2,5 M Butyllithiumlösung in Hexan versetzt und 1 h zum Rückfluß erhitzt. Die so erhaltene Lösung wurde bei Raumtemperatur zu einer Lösung von 4,1 g (32 mmol) Dimethyldichlorsilan in 20 ml THF getropft und 3 h zum Rückfluß erhitzt. Die Reaktionsmischung wurde auf Eiswasser gegossen und mehrmals ausgeethert. Die vereinigten organischen Phasen wurden über Natriumsulfat getrocknet und unter vermindertem Druck eingedampft. Der Rückstand wurde an 450 g Kieselgel 60 chromatographiert. Mit einem Laufmittelgemisch aus Hexan/5 % Methylenchlorid ließen sich zunächst 2,5 g des Indens 2 eluieren. Mit Hexan/8 % Methylenchlorid folgten 6,5 g des Ligandsystems 7 (Isomere). Die Ausbeute betrug 54 % bzw. 72 % bezüglich umgesetztem Inden 2.

### Beispiel J

### Dimethylsilandiylbis(2,4,6-trimethylindenyl)zirkondichlorid (8)

Eine Lösung von 2,0 g (5,4 mmol) des Ligandsystems 7 in 30 ml Diethylether wurde bei Raumtemperatur unter Ar-Schutz mit 6,6 ml (16,2 mmol) einer 2,5 M Butyllithiumlösung in Hexan versetzt und 5-6 h bei dieser Temperatur gerührt. Die Lösung wurde vollständig eingedampft. Der verbleibende feste Rückstand wurde portionsweise mit insgesamt 30 ml Hexan gewaschen und lange im Ölpumpenvakuum getrocknet. Das so erhaltene beigefarbene Pulver wurde bei - 78°C zu einer Suspension von 1,23 g (5,5 mmol) Zirkontetrachlorid in 30 ml Methylenchlorid gegeben. Nach dem Aufwärmen auf Raumtemperatur wurde die Reaktionsmischung vollständig eingedampft und im Ölpumpenvakuum getrocknet. Der feste Rückstand bestand aus einer Mischung der racemischen mit der meso-Form im Verhältnis 1:1. Dieser wurde zunächst mit einer kleinen Menge Hexan gewaschen. Anschließend wurde mit insgesamt 120 ml Toluol extrahiert. Die Lösung wurde eingeengt und bei -35°C der Kristallisation überlassen. Es ließen sich 800 mg (28 %) des Zirkonocens 8 als reines Racemat in Form orangefarbener Kristalle gewinnen.
¹H-NMR-Spektrum des Racemats (100 MHz, CDCl₃): 7,20 (s,2,Arom.-H), 6,97 (s,2,Arom.-H), 6,70 (s,2,β-Ind-H), 2,32 (s,6,CH₃), 2,27 (s,6,CH₃), 2,20 (s,6,CH₃), 1,27 (s,6,Si-CH₃).
Massenspektrum: 530 M⁺ (bzgl. ⁹⁰Zr), korrektes Isotopenmuster, korrekter Zerfall.

### Beispiel K

### Methylphenylbis(2-methyl-4,6-diisopropylindenyl)silan (9).

Eine Lösung von 10 g (46 mmol) des Indens 4/4a in 200 ml THF wurde unter Ar-Schutz bei Raumtemperatuzr mit 18,6 ml (46 mmol) einer 2,5 M Butyllithiumlösung in Hexan versetzt und 1 h zum Rückfluß erhitzt. Die Lösung wurde bei Raumtemperatur zu einer Lösung von 4,48 g (23 mmol) Methylphenyldichlorsilan in 30 ml THF getropft und 3 h zum Rückfluß erhitzt. Die Mischung wurde auf Eiswasser gegossen und mehrmals ausgeethert. Die vereinigten organischen Phasen wurden mit Natriumsulfat getrocknet und eingedampft. Der Rückstand wurde an 450 g Kieselgel 60 chromatographiert. Mit einem Laufmittelgemisch aus Hexan/Methylenchlorid (10:1) konnten zunächst 1,9 g nicht umgesetztes Inden 4/4a zurückgewonnen werden. Anschließend folgten 7,4 g des Ligandsystems 9 (Isomerengemsich). Die Ausbeute betrug 57 % bzw. 73 % bezüglich umgesetztem Inden.

### Beispiel L

### Methylphenylsilylbis(2-methyl-4,6-diisopropylindenyl)zirkondichlorid (10)

Eine Lösung von 7,4 g (13,5 mmol) des Ligandsystems 9 in 30 ml Diethylether wurde bei Raumtemperatur unter Ar-Schutz mit 11,2 ml (28 mmol) einer 2,5 M Butyllithiumlösung in Hexan versetzt und 16 h bei Raumtemperatur gerührt. Nach dem Abziehen des Lösemittels wurde der verbleibende Rückstand 3-4 h bei 40-50°C getrocknet und anschließend bei -78°C zu einer Suspension von 3,2 g (13,5 mmol) Zirkontetrachlorid in 40 ml Methylenchlorid gegeben. Nach dem Aufwärmen auf Raumtemperatur wurde das Lösemittel unter vermindertem Druck abgezogen. Der verbleibende feste Rückstand wurde im Ölpumpenvakuum getrocknet und mit 100 ml Hexan extrahiert. Nach Abziehen des Lösemittels erhält man 5,4 g (55 %) des Zirkonocens 10 als Mischung der racemischen mit der meso-Form im Verhältnis 2:1 (orangebraunes Kristallpulver). Durch Umkristallisation aus Hexan ist die reine racemische Form erhältlich.
¹H-NMR-Spektrum der Isomerenmischung (100 MHz, CDCl₃): 6,6-8,2 (m, Arom.-H,ß-Ind-H), 2,5-3,2 (m,i-Pr-H), 2,52 (s,CH₃), 2,32 (s,CH₃), 2,20 (s,CH₃), 1,90 (s,CH₃), 1,0-1,5 (m,i-Pr-CH₃,Si-CH₃).
Massenspektrum: 704 M⁺ (bzgl. ⁹⁰Zr), korrektes Isotopenmuster, korrekter Zerfall.

### Beispiel M

### 1,2-Bis(2-methyl-4,6-diisopropylindenyl)ethyl (11)

Eine Lösung von 5,0 g (23,3 mmol) des Indens 4/4a in 50 ml THF wurde unter Ar-Schutz bei Raumtemperatur mit 18,6 ml (46 mmol) einer 2,5 M Butyllithiumlösung in Hexan versetzt und 1 h zum Rückfluß erhitzt. Die Lösung wurde bei -78°C zu einer Lösung von 2,18 g (11,0 mmol) 1,2-Dibromethan gegeben. Die Lösung wurde langsam auf Raumtemperatur erwärmt und über Nacht bei dieser Temperatur gerührt. Die Mischung wurde auf Eiswasser gegossen und mehrmals ausgeethert. Die vereinigten organischen Phasen wurden mit Natriumsulfat getrocknet und eingedampft. Der Rückstand wurde an 450 g Kieselgel 60 chromatographiert. Mit einem Laufmittelgemisch aus Hexan/Methylenchlorid (20:1 bis 10:1) konnten zunächst 1,2 g nicht umgesetztes Inden 4/4a zurückgewonnen werden. Anschließend folgten 1,7 g des Ligandsystems 11 (farbloser Feststoff). Die Ausbeute betrug 35 % bzw. 45 % bezüglich umgesetztem Inden.

### Beispiel N

### 1,2-Ethandiylbis(2-methyl-4,6-diisopropylindenyl)zirkondichlorid (12)

Eine Lösung von 1,6 g (3,52 mmol) des Ligandsystems 11 in 20 ml Diethylether wurde bei Raumtemperatur unter Ar-Schutz mit 3,5 ml (8,8 mmol) einer 2,5 M Butyllithiumlösung in Hexan versetzt und über Nacht gerührt. Der nach Abziehen des Lösemittels verbleibende Rückstand wurde mit Hexan gewaschen und lange im Ölpumpenvakuum getrocknet. Das so erhaltene Pulver wurde bei -78°C zu einer Suspension von 815 mg (3,5 mmol) Zirkontetrachlorid in 15 ml Methylenchlorid gegeben. Nach dem Erwärmen auf Raumtemperatur wurde noch 1 h gerührt und das Lösemittel unter vermindertem Druck entfernt. Der im Ölpumpenvakuum getrocknete Rückstand wurde mit Toluol extrahiert. Nach Abziehen des Lösemittels und Waschen mit Hexan erhielt man 1,5 g (70 %) des Zirkonocens 18 als Mischung der racemischen mit de meso-Form im Verhältnis 2:1 (orangefarbenes Pulver). Durch Umkristallisation aus einem Toluol/Hexan-Gemisch ließen sich 700 mg (32 %) des reinen Racemats gewinnen.
¹H-NMR-Spektrum des Racemats (100 MHz, CDCl₃): 7,3 (s,Arom.-H), 7,0 (s,Arom.-H), 6,55 (s,ß-Ind-H), 3,6 (s,C₂H₄), 2,6-3,2 (m,i-Pr-H), 2,2 (s,CH₃), 1,0-1,5 (m,i-Pr-CH₃)
Massenspektrum: 612 M⁺ (bzgl. ⁹⁰Zr), korrektes Isotopenmuster, korrekter Zerfall.

### Beispiel O

### 1,2-Bis(2-methyl-4,6-diisopropylindenyl)butan (13)

Eine Lösung von 5,0 g (23,3 mmol) des Indens 4/4a wurde analog Beispiel M mit 2,37 g (11 mmol) 1,2-Dibrombutan (97 %) umgesetzt. Die Chromatographie an Kieselgel lieferte mit Hexan als Laufmittel nach Edukt und einem Nebenprodukt (Spiroverbindung) 1,16 g (22 %) des Liganden 13 als Isomerengemisch. Die Isomere konnten durch eine nochmalige Chromatographie an einer langen Säule getrennt bzw. angereichert werden.

### Beispiel P

### rac-1,2-Butandiylbis(2-methyl-4,6-diisopropylindenyl)zirkondichlorid (14)

Eine Lösung von 1,0 g (2,07 mmol) des Ligandsystems 13 (2 Isomere) in 15 ml Diethylether wurde analog Beispiel N umgesetzt. Die Umkristallisation aus Toluol/Hexan-Mischungen lieferte bei -35°C insgesamt 1,24 g (60 %) des Metallocens 14 als Mischung der verschiedenen Diastereomere der racemischen und der meso-Form. Durch nochmalige Umkristallisation konnte das Racemat 14 als 2 Diastereomere erhalten werden.
¹H-NMR-Spektrum (100 MHz, CDCl₃): 7,0-7,4 (m,Aromaten-H) 6,5 und 6,6 (s,ß-Ind-H), 3,3-3,7 (m,C₂H₃), 1,0-2,8 (m,CH₃,i-Pr, C₂H₅). Massenspektrum: 640 M⁺, korrektes Isotopenmuster, korrekter Zerfall.

### Polymerisationsbeispiele:

### Beispiel I

Ein trockener 24 dm³-Reaktor wurde mit Propylen gespült und mit 12 dm³ flüssigem Propylen befüllt. Dann wurden 35 cm³ toluolische Methylaluminoxanlösung (entsprechend 52 mmol Al, mittlerer Oligomerisierungsgrad p = 20) zugegeben und der Ansatz bei 30°C 15 Minuten gerührt.

Parallel dazu wurden 3,5 mg (0,005 mmol) rac-Dimethylsilyl (2-methyl-4,6-diisopropyl-1-indenyl)₂ zirkondichlorid in 13,5 cm³ toluolischer Methylaluminoxanlösung (20 mmol Al) gelöst und durch 15 minütiges Stehenlassen voraktiviert.

Die weinrote Lösung wurde dann in den Reaktor gegeben, durch Wärmezufuhr auf 75°C aufgeheizt (10°C/min) und das Polymerisationssystem 1 h durch Kühlung bei 75°C gehalten. Gestoppt wurde die Polymerisation durch Abgasen des überschüssigen Monomeren. Es wurden 2,11 kg Polypropylen erhalten.
Die Aktivität des Metallocens betrug somit 603 kgPP/g Metallocen x h.
VZ = 259 cm³/g, M_{w} = 305 000 g/mol; M_{w}/Mₙ = 2,0;
II = 96,0 %, MFI (230/5) = 8,5 dg/min.

### Vergleichsbeispiel 1

Beispiel 1 wurde mit dem Metallocen rac-Dimethylsilyl(2-methyl-1-indenyl)₂ zirkondichlorid wiederholt. Die Metallocenaktivität war 395 kg PP/g Metallocen x h, VZ = 159 cm³/g, M_{w} = 158 000 g/mol, M_{w/}Mₙ = 2,1 und der MFI (230/5) war 48 dg/min. Der isotaktische Index (II) betrug 96,0 %.

### Vergleichsbeispiel 2

Beispiel 1 wurde mit dem Metallocen rac-Dimethylsilyl(2-methyl-4-isopropyl-1-indenyl)₂zirkondichlorid wiederholt. Die Metallocenaktivität war 460 kg PP/g Metallocen x h, VZ = 152 cm³/g, M_{w} = 147 500 g/mol, M_{w}/Mₙ = 2,3 und MFI (230/5) = 51 dg/min.

### Vergleichsbeispiel 3

Beispiel 1 wurde mit dem Metallocen rac-Dimethylsilyl(1-indenyl)₂zirkondichlorid wiederholt. Die Metallocenaktivität war 695 kg PP/g Metallocen x h, VZ = 31 cm³/g, M_{w} = 18 500 g/mol, M_{w}/Mₙ = 2,2, MFI (230/5) war nicht mehr meßbar.

### Vergleichsbeispiel 4

Beispiel 1 wurde mit dem Metallocen rac-Dimethylsilyl(4,7-dimethyl-1-indenyl)₂ zirkondichlorid wiederholt. Die Metallocenaktivität war 195 kg PP/g Metallocen x h, VZ = 16 cm³/g, M_{w} = 9 500 g/mol, M_{w}/Mₙ = 2,0, II = 87 %, der MFI (230/5) war nicht mehr meßbar.

Die vier Vergleichsversuche zeigen, daß mit den am Indenylliganden in unterschiedlicher Weise substituierten Metallocenen bzw. mit dem unsubstituierten Metallocen hergestellte Polypropylene deutliche Molmassenunterschiede aufweisen. Unter Einbeziehung des erfindungsgemäßen Metallocens aus Beispiel 1 reicht die Spanne vom Wachsbereich (Vergleichsbeispiel 4) bis zum sehr hochmolekularen erfindungsgemäßen Polymer (Beispiel 1).
Diese Versuche belegen die Überlegenheit der in 2,4,6-Stellung substituierten Metallocene.

### Vergleichsbeispiel 5

Beispiel 1 wurde mit dem Metallocen rac-Dimethylsilyl(3-methyl-1-indenyl)₂ zirkondichlorid wiederholt.
Es wurde ein Polypropylen mit nicht akzeptablem isotaktischen Index und mit niedriger Molmasse erhalten.

### Beispiel 2

Beispiel 1 wurde mit 5,1 mg (0,008 mmol) des Metallocens rac-Dimethylsilyl(2-methyl-4,6-diisopropyl-1-indenyl)₂zirkondichlorid wiederholt. Die Polymerisationstemperatur war 50°C. Es wurden 0,85 kg Polypropylen, entsprechend einer Metallocenaktivität von 166,7 kg PP/g Metallocen x h erhalten. VZ = 454 cm³/g, M_{w} = 498 500 g/mol, M_{w}/Mₙ = 2,2, II = 97,1 %, MFI (230/5) = 1,7 dg/min.

### Beispiel 3

Beispiel 1 wurde mit 4,5 mg (0,007 mmol) des Metallocens rac-Dimethylsilyl(2-methyl-4,6-diisopropyl-1-indenyl)₂-zirkondichlorid bei 60°C Polymerisationstemperatur wiederholt. Es wurden 1,34 kg Polypropylen, entsprechend einer Metallocenaktivität von 298 kg PP/g Metallocen x h erhalten. VZ = 347 cm³/g; M_{w} = 444500 g/mol, M_{w}/Mₙ = 2,1; II = 97,0 %; MFI (230/5) = 3,2 dg/min, Schmp. = 145°C.

### Beispiel 4

Beispiel 1 wurde mit 9,6 mg (0,015 mmol) des Metallocens rac-Dimethylsilyl(2-methyl-4,6-diisopropyl-1-indenyl)₂-zirkondichlorid bei 30°C Polymerisationstemperatur wiederholt. Diese Polymerisationstemperatur ist zwar großtechnisch weniger geeignet, der Versuch belegt jedoch das Molmassenpotential und die hohe Aktivität des Metallocens.
Es wurden 0,61 kg Polypropylen, entsprechend einer Metallocenaktivität von 63,5 kg PP/g Metallocen x h erhalten.
VZ = 645 cm³/g; M_{w} = 867000 g/mol, M_{w}/Mₙ = 2,1 ; II = 97,7 %; MFI (2320/5) = 0,26 dg/min.

### Beispiel 5

Beispiel 1 wurde mit 3,3 mg (0,006 mmol) des Metallocens rac-Dimethylsilyl(2,4,6-trimethyl-1-indenyl)₂zirkondichlorid wiederholt. Es wurden 1,83 kg Polypropylen erhalten. Die Metallocenaktivität war somit 555 kg PP/g Metallocen x h.
VZ = 165 cm³/g, M_{w} = 186 000 g/mol; M_{w}/Mₙ = 2,0; Schmp. = 145°C; MFI (230/5) = 40 dg/min.

### Beispiel 6

Beispiel 1 wurde mit 4,4 mg (0,006 mmol) des Metallocens rac-Phenyl(Methyl)silyl(2-methyl-4,6-diisopropyl-l-indenyl)₂zirkondichlorid bei 70°C Polymerisationstemperatur wiederholt. Es wurden 2,05 kg Polypropylen, entsprechend einer Metallocenaktivität von 466 kg PP/g Metallocen x h erhalten.
VZ = 263 cm³/g, M_{w} = 385 500 g/mol, M_{w}/Mₙ = 2,5, Schmp. = 145°C, MFI (230/5) = 8,5 dg/min.

### Beispiel 7

Beispiel 6 wurde mit 7,9 mg (0,011 mmol) des Metallocens bei 50°C Polymerisationstemperatur wiederholt. Die Metallocenaktivität war 156 kg PP/g Metallocen x h.
VZ = 498 cm³/g, M_{w} = 586 000 g/mol, M_{w}/Mₙ = 3.0, Schmp. = 147°C, MFI (230/5) = 1,5 dg/min.

### Beispiel 8

Beispiel 6 wurde mit 12,0 mg (0,017 mmol) des Metallocens bei 30°C Polymerisationstemperatur wiederholt. Die Metallocenaktivität war 58,3 kg PP/g Metallocen x h.
VZ = 811 cm³/g, M_{w} = 1 020 000 g/mol, M_{w}/Mₙ = 2,3, Schmp. = 148°C, MFI (230/5) = 0,2 dg/min.

### Beispiel 9

Beispiel 7 wurde mit 2,8 mg des Metallocens wiederholt. In den Reaktor wurde vor der Polymerisation 24 Ndm³ Wasserstoff eindosiert. Die Metallocenaktivität war 600 kg PP/g Metallocen x h.
VZ = 30 cm³/g, M_{w} = 18 250 g/mol, M_{w}/Mₙ = 2,5, Schmp. = 144°C.

### Beispiel 10

Beispiel 7 wurde mit 3,5 mg des Metallocens und mit 60 Ndm³ Wasserstoff wiederholf. Die Metallocenaktivität war 650 kg PP/g Metallocen x h.
VZ = 14 cm³/g, M_{w} = 6300 g/mol, M_{w}/Mₙ = 2,2, Schmp. = 145°C.

Die Beispiele 9 und 10 belegen die excellente Wasserstoffansprechbarkeit des Metallocens zur Einstellung einer gewünschten Molmasse. Mit geringen Mengen Wasserstoff läßt sich die Kettenlänge in weiten Grenzen bis in den Wachsbereich variieren.

### Beispiel 11

Ein trockener 150 dm³-Reaktor wurde mit Stickstoff gespült und bei 20° C mit 80 dm³ eines entaromatisierten Benzinschnittes mit dem Siedebereich 100-120°C gefüllt. Dann wurde der Gasraum durch 5-maliges Aufdrücken von 2 bar Propylen und Entspannen stickstofffrei gespült.
Nach Zugabe von 50 I flüssigem Propylen wurden 64 cm³ toluolische Methylaluminoxanlösung (entsprechend 100 mmol Al, Molmasse nach kryoskopischer Bestimmung 1050 g (mol)) zugegeben und der Reaktorinhalt auf 50°C aufgeheizt. Durch Zudosierung von Wasserstoff wurde ein Wasserstoffgehalt im Gasraum des Reaktors von 0,2 % eingestellt und später dann durch Nachdosierung während der gesamten Polymerisationszeit konstant gehalten (Überprüfung on-Line durch Gaschromatographie).
15,5 mg rac-Phenyl(Methyl)silyl(2-methyl-4,6-diisopropyl-l-indenyl)₂zirkondichlorid wurden in 32 ml toluolischer Methylaluminoxanlösung (entsprechend 50 mmol Al) gelöst und nach 15 Minuten in den Reaktor gegeben.

Durch Kühlung wurde der Reaktor 18 h bei 50°C Polymeristionstemperatur gehalten, dann wurde durch Zugabe von 2 bar CO₂-Gas die Polymerisation gestoppt und das gebildete Polymere auf einer Druckmutsche vom Suspensionsmedium abgetrennt. Die Trocknung des Produktes erfolgte 24 h bei 80°C/200 mbar. Es wurden 20,9 kg Polymerpulver, entsprechend einer Metallocenaktivität von 74,9 kg PP/g Metallocen x h erhalten.
VZ = 424 cm³/g, M_{w} = 518 000 g/mol, M_{w}/Mₙ = 2,0, Smp. = 149°C, MFI (230/5) = 4,1 dg/min.

### Beispiel 12

Ein trockener 24 dm³-Reaktor wurde mit Propylen gespült und mit 2,4 Ndm³ Wasserstoff und 12 dm³ flüssigem Propylen befüllt. Dann wurden 35 cm³ toluolische Methylaluminoxanlösung (entsprechend 52 mmol Al, mittlerer Oligomerisierungsgrad p = 20) zugegeben.
Parallel dazu wurden 6,5 mg rac-Phenyl(Methyl)-silyl(2-methyl-4,6-diisopropyl-1-indenyl)zirkondichlorid in 13,5 cm³ toluolischer Methylaluminoxanlösung (20 mmol Al) gelöst und durch 5 minütiges Stehenlassen voraktiviert.
Die Lösung wurde dann in den Reaktor gegeben und unter kontinuierlicher Zugabe von 60 g Ethylen wurde 1 h bei 60°C polymerisiert. Die Metallocenaktivität betrug 398 kg PP/g Metallocen x h. Der Ethylengehalt des Copolymeren betrug 2,0 %.
VZ = 503 cm³/g, M_{w} = 384 000 g/mol, M_{w}/Mₙ = 2,0, Smp. = 139°C, laut NMR-Spektroskopie wurde das Ethylen überwiegend isoliert (statistisches Copolymer) eingebaut.

### Beispiel 13

Ein trockener 150 dm³-Reaktor wurde wie in Beispiel 11 befüllt.
18,9 mg rac-Phenyl(Methyl)silyl(2-methyl-4,6-diisopropyl-1-indenyl)₂zirkondichlorid wurden in 32 ml toluolischer Methylaluminoxanlösung (50 mmol) gelöst und in den Reaktor gegeben.
Die Polymerisation erfolgte in einer 1. Stufe bei 70°C 5 Stunden lang. In einer 2. Stufe wurden dann bei 55°C 3 kg Ethylen schnell zugegeben und nach weiteren 3 h Polymerisation bei 55°C wurde mit CO₂-Gas gestoppt. Es wurden 25,9 kg Blockcopolymerpulver erhalten.
VZ = 344 cm³/g, M_{w} = 399 000 g/mol, M_{w}/Mₙ = 3,8, MFI (230/5) = 5,0 dg/min.

Das Blockcopolymer enthielt 10,8 Gew.-% Ethylen. Die Fraktionierung ergab einen Gehalt von 27,5 Gew.-% Ethylen/Propylen-Kautschuk. Die Glastemperatur des Kautschuks war -51°C.

### Beispiel 14

Beispiel 1 wurde bei 70°C Polymerisationstemperatur mit 4,0 mg des Metallocens rac-1,2-Ethandiylbis(2-methyl-4,6-diisopropyl-1-indenyl)zirkondichlorid wiederholt.
Die Metallocenaktivität war 529 kg PP/g Metallocen x h.
VZ = 149 cm³/g, M_{w} = 174 500 g/mol, M_{w}/Mₙ = 1,9, Schmp. = 141 °C, MFI (230/5) = 74 dg/min.

### Beispiel 15

Beispiel 14 wurde mit 4,0 mg des Metallocens rac-1,2-Butandiylbis(2-methyl-4,6-diisopropyl-1-indenyl)zirkondichlorid wiederholt. Die Metallocenaktivität war 319 kgPP/g Metallocen x h.
VZ = 295 cm³/g, M_{w} = 368 500 g/mol, M_{w}/Mₙ = 2,1, Schmp. = 142°C, MFI (230/5) = 4,0 dg/min.

## Patentansprüche

1. Verwendung eines Indens der Formel VII oder VIIa 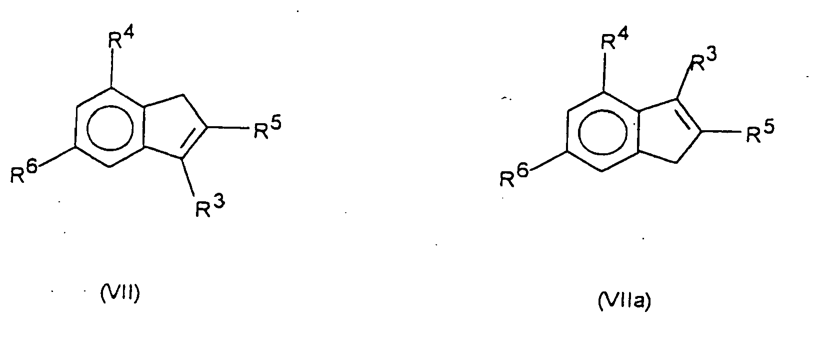 zur Herstellung eines Metallocens der Formel I 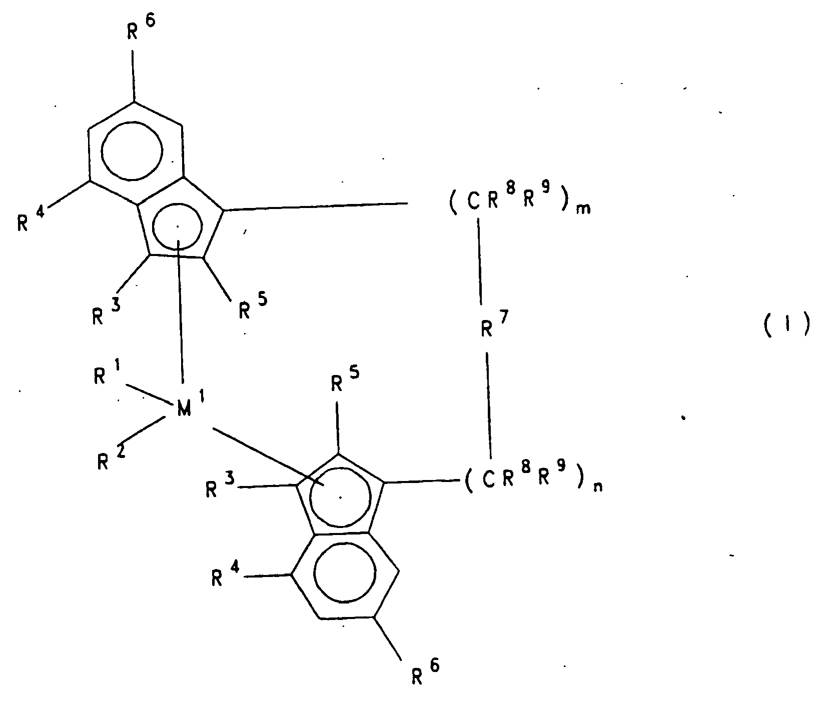 worin
M¹ ein Metall der Gruppe IVb, Vb oder Vlb des Periodensystems ist,
R¹ und R² gleich oder verschieden sind und ein Wasserstoffatom, eine C₁-C₁₀-Alkylgruppe, eine C₁-C₁₀-Alkoxygruppe, eine C₆-C₁₀₋Arylgruppe, eine C₆-C₁₀-Aryloxygruppe, eine C₂-C₁₀-Alkenylgruppe, eine C₇-C₄₀-Arylalkylgruppe, eine C₇-C₄₀-Alkylarylgruppe, eine C₈-C₄₀-Arylalkenylgruppe oder ein Halogenatom bedeuten,
die Reste R³ Wasserstoff bedeuten,
R⁴ bis R⁶ gleich oder verschieden sind und ein
Halogenatom, eine C₁-C₁₀-Alkylgruppe, die halogeniert sein kann,
eine C₆-C₁₀-Arylgruppe, die halogeniert sein kann, einen -NR₂¹⁰, -SR¹⁰, -OSiR₃¹⁰, -SiR₃¹⁰ oder -PR₂¹⁰-Rest bedeuten, worin R¹⁰ ein Halogenatom, eine C₁-C₁₀-Alkylgruppe oder eine C₆-C₁₀-Arylgruppe ist,
R⁷ 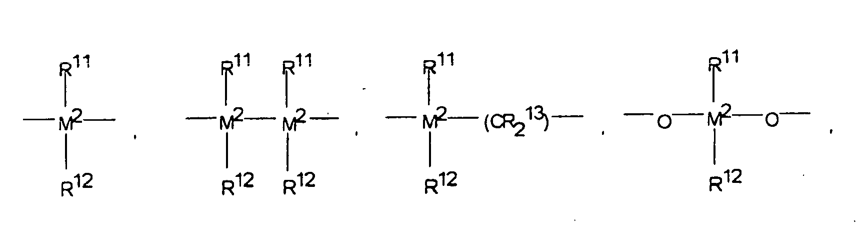 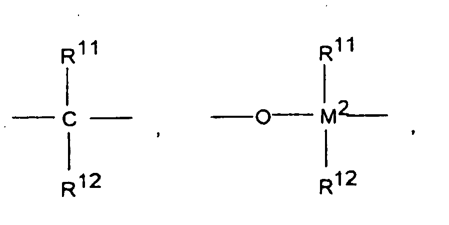 =BR¹¹, =AlR¹¹, -Ge-, -Sn-, -O-, -S-, =SO, =SO₂, =NR¹¹, =CO, =PR¹¹ oder =P(O)R¹¹ ist,
wobei
R¹¹, R¹² und R¹³ gleich oder verschieden sind und ein Wasserstoffatom, ein Halogenatom, eine C₁-C₁₀-Alkylgruppe, C₁-C₁₀-Fluoralkylgruppe, eine C₆₋C₁₀-Arylgruppe, eine C₆-C₁₀-Fluorarylgruppe, eine C₁-C₁₀-Alkoxygruppe, eine C₂-C₁₀-Alkenylgruppe, eine C₇-C₄₀-Arylalkylgruppe, eine C₈-C₄₀₋Arylalkenylgruppe, eine C₇-C₄₀-Alkylarylgruppe bedeuten oder R¹¹ und R¹² oder R¹¹ und R¹³ jeweils mit den sie verbindenden Atomen einen Ring bilden,
M² Silizium, Germanium oder Zinn ist,
R⁸ und R⁹ gleich oder verschieden sind und die für R¹¹ genannte Bedeutung haben und
m und n gleich oder verschieden sind und null, 1 oder 2 sind, wobei m plus n null, 1 oder 2 ist.

2. Verfahren zur Herstellung eines Indens der Formel VII oder VIIa 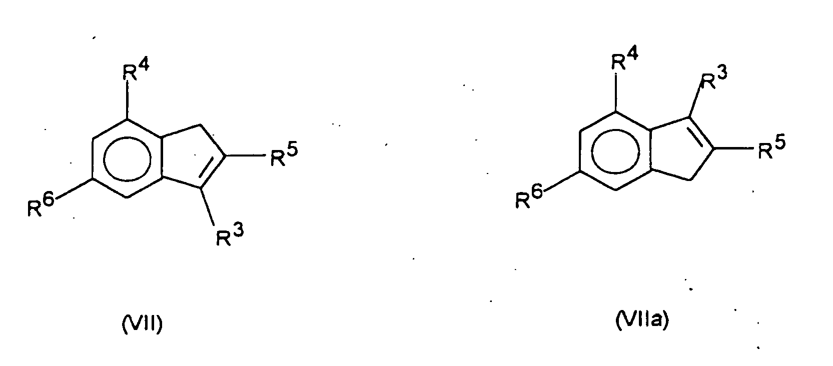 wobei
a) eine Verbindung IV 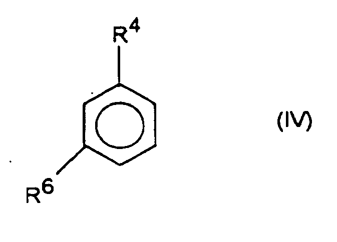 mit einer Verbindung V 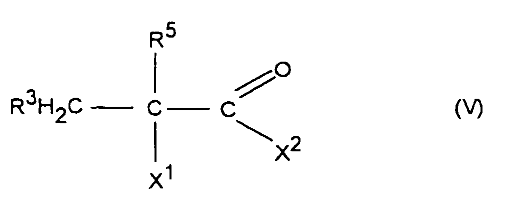 oder deren Anhydrid in Gegenwart eines Friedel-Crafts-Katalysators umgesetzt wird zu einem Indanon VI bzw. VIa 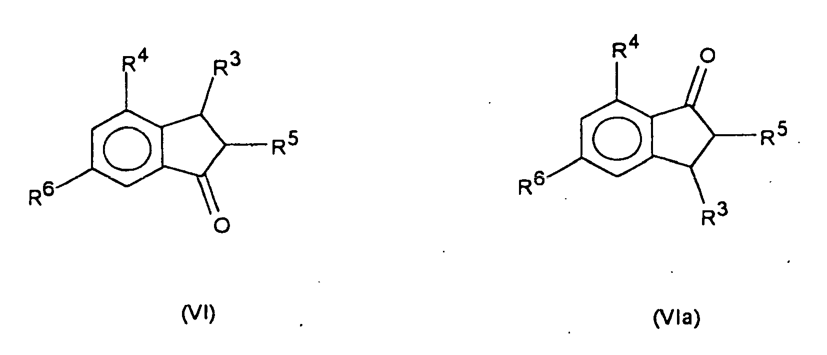 wobei in den Formel IV,V, VI, VIa, VII und VIIa die Reste R³ bis R⁶ wie in Formel I definiert sind und X¹ und X² für eine nucleophile Abgangsgruppe stehen, und
b) das Indanon anschließend mit einem Reduktionsmittel zu dem entsprechenden Indanol reduziert wird und dann dehydratisiert wird.

3. Inden der Formel VII oder VIIa 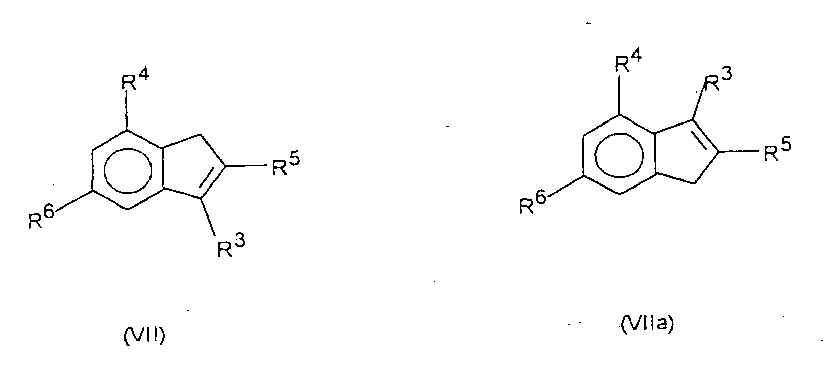 worin
R³ ein Wasserstoffatom ist,
R⁴ bis R⁶ gleich oder verschieden sind und ein Halogenatom, eine C₁-C₁₀₋Alkylgruppe, die halogeniert sein kann, eine C₆-C₁₀-Arylgruppe, die halogeniert sein kann, einen -NR¹⁰₂, -SR¹⁰, -OSiR¹⁰₃, -SiR¹⁰₃ oder -PR¹⁰₂-Rest bedeuten, worin R¹⁰ ein Halogenatom, eine C₁-C₁₀-Alkylgruppe oder ein C₆-C₁₀₋Arylgruppe ist, wobei 2,4,6-Trichlorinden ausgeschlossen ist.

4. Indanon der Formel Vla 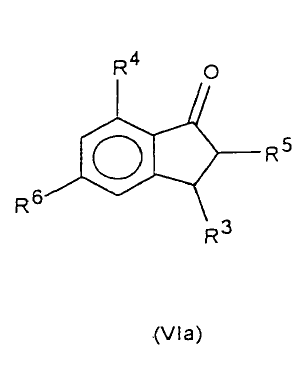 worin
R³ ein Wasserstoffatom, ein Halogenatom, eine C₁-C₁₀-Alkylgruppe, die halogeniert sein kann, eine C₆-C₁₀-Arylgruppe, die halogeniert sein kann, einen -NR₂¹⁰, -SR¹⁰, -OSiR₃¹⁰, -SiR₃¹⁰ oder -PR₂¹⁰-Rest bedeutet, worin R¹⁰ ein Halogenatom, eine C₁-C₁₀-Alkylgruppe oder eine C₆-C₁₀-Arylgruppe ist,
R⁴ bis R⁶ gleich oder verschieden sind und die für R³ genannte Bedeutung haben, mit der Maßgabe, daß R⁴ und R⁶ kein Wasserstoffatom sind.

5. Indanon gemäß Anspruch 4, worin R³ ein Wasserstoffatom ist und R⁵ kein Wasserstoffatom ist.

6. 2-Methyl-4,6-diisopropyl-1-indanon.

7. Verwendung eines Indanons gemäß Anspruch 4 bis 6 zur Herstellung eines Indens wie in Anspruch 3 definiert.

## Claims

1. Use of an indene of the formula VII or VIIa 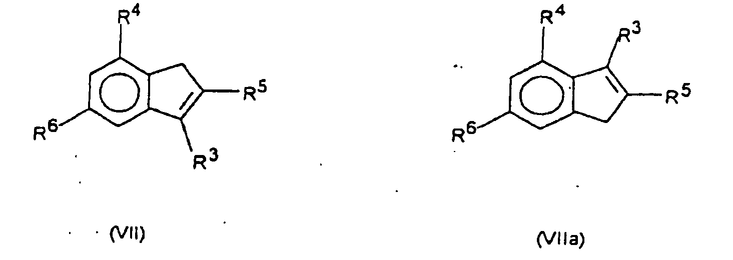 for preparing a metallocene of the formula I 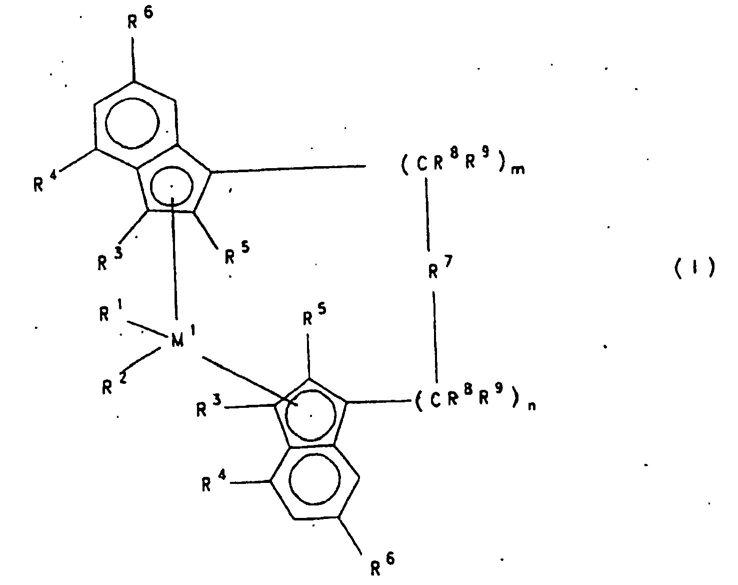 in which
M¹ is a metal of group IVb, Vb or VIb of the periodic table,
R¹ and R ² are identical or different and are a hydrogen atom, a C₁-C₁₀-alkyl group, a C₁-C₁₀₋alkoxy group, a C₆-C₁₀-aryl group, a C₆-C₁₀₋aryloxy group, a C₂-C₁₀-alkenyl group, a C₇-C₄₀-arylalkyl group, a C₇-C₄₀-alkylaryl group, a C₈-C₄₀-arylalkenyl group or a halogen atom,
the radicals R³ are hydrogen,
R⁴ to R⁶ are identical or different and are a halogen atom, a C₁-C₁₀-alkyl group, which can be halogenated, a C₆-C₁₀-aryl group, which can be halogenated, an -NR₂¹⁰, -SR¹⁰, -OSiR₃¹⁰, -SiR₃¹⁰ or -PR₂¹⁰ radical, in which R¹⁰ is a halogen atom, a C₁-C₁₀-alkyl group or a C₆-C₁₀-aryl group,
R⁷ is 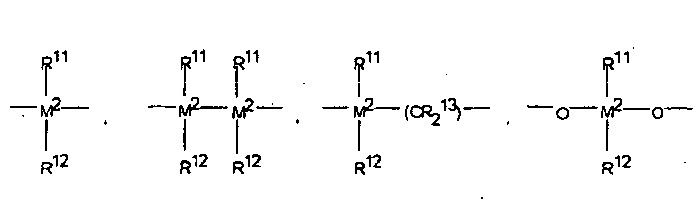 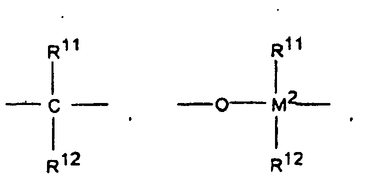 =BR¹¹, =AlR¹¹, -Ge-, -Sn-, -O-, -S-, =SO, =SO₂, =NR¹¹, =CO, =PR¹¹ or =P(O)R¹¹,
wherein
R¹¹, R¹² and R¹³ are identical or different and are a hydrogen atom, a halogen atom, a C₁-C₁₀-alkyl group, C₁-C₁₀-fluoroalkyl group, a C₆-C₁₀-aryl group, a C₆-C₁₀-fluoroaryl group, a C₁-C₁₀-alkoxy group, a C₂-C₁₀-alkenyl group, a C₇-C₄₀-arylalkyl group, a C₈-C₄₀-arylalkenyl group, a C₇-C₄₀₋alkylaryl group, or R¹¹ and R¹², or R¹¹ and R¹³, in each case with the atoms joining them, form a ring,
M² is silicon, germanium or tin,
R⁸ and R⁹ are identical or different and have the meaning given for R¹¹ and
m and n are identical or different and are zero, 1 or 2, m plus n being zero, 1 or 2.

2. Process for preparing an indene of the formula VII or VIIa 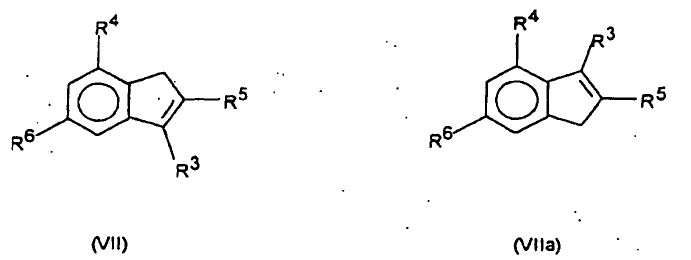 wherein
a) a compound IV 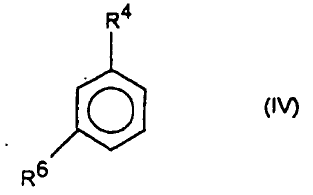 is reacted with a compound V 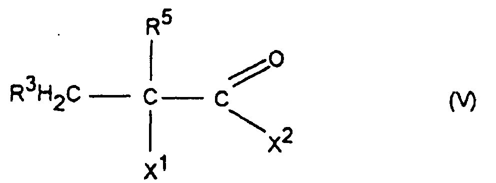 or its anhydride in the presence of a Friedel-Crafts catalyst to form an indanone VI or VIa 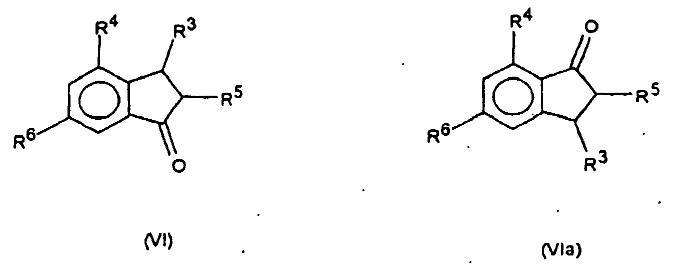 wherein, in the formula IV, V, VI, VIa, VII and VIIa, the radicals R³ to R⁶ are as defined in the formula I and X¹ and X² are a nucleophilic leaving group, and
b) the indanone is subsequently reduced to the corresponding indanol using a reducing agent and then dehydrated.

3. Indene of the formula VII or VIIa 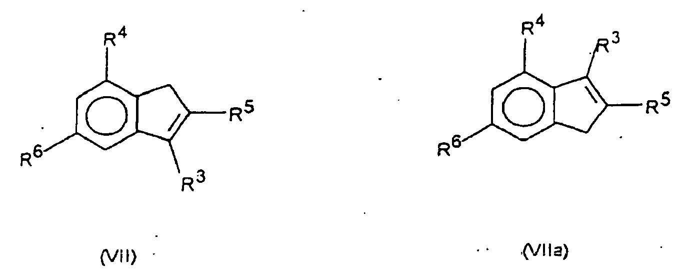 in which
R³ is a hydrogen atom,
R⁴ to R⁶ are identical or different and are a halogen atom, a C₁-C₁₀-alkyl group, which can be halogenated, a C₆-C₁₀-aryl group, which can be halogenated, an -NR¹⁰₂, -SR¹⁰, -OSiR¹⁰₃, -SiR¹⁰₃ or -PR¹⁰₂ radical, in which R¹⁰ is a halogen atom, a C₁-C₁₀-alkyl group or a C₆-C₁₀-aryl group, excluding 2,4,6-trichloroindene.

4. Indanone of the formula VIa 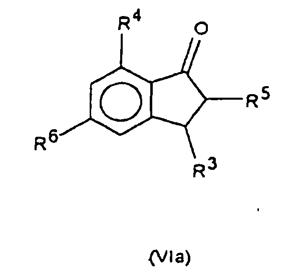 in which
R³ is a hydrogen atom, a halogen atom, a C₁-C₁₀- alkyl group, which can be halogenated, a C₆-C₁₀-aryl group, which can be halogenated, an -NR₂¹⁰, -SR¹⁰, -OSiR₃¹⁰, -SiR₃¹⁰ or -PR₂¹⁰ radical, in which R¹⁰ is a halogen atom, a C₁-C₁₀-alkyl group or a C₆-C₁₀-aryl group,
R⁴ to R⁶ are identical or different and have the meaning given for R³, with the proviso that R⁴ and R⁶ are not a hydrogen atom.

5. Indanone according to Claim 4 wherein R³ is a hydrogen atom and R⁵ is not a hydrogen atom.

6. 2-Methyl-4,6-diisopropyl-1-indanone.

7. Use of an indanone according to Claim 4 to 6 for preparing an indene as defined in Claim 3.

## Revendications

1. Utilisation d'un indène de formule VII ou VIIa 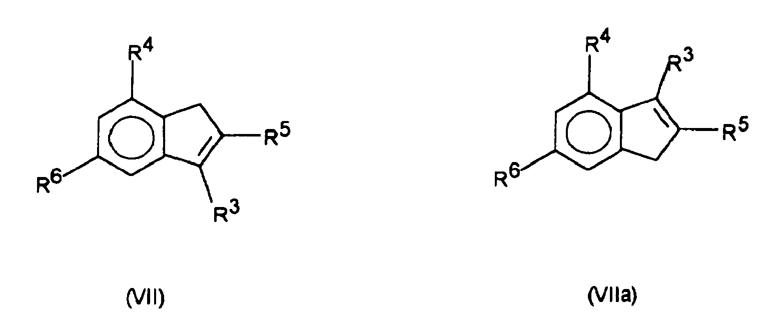 pour la préparation d'un métallocène de formule I 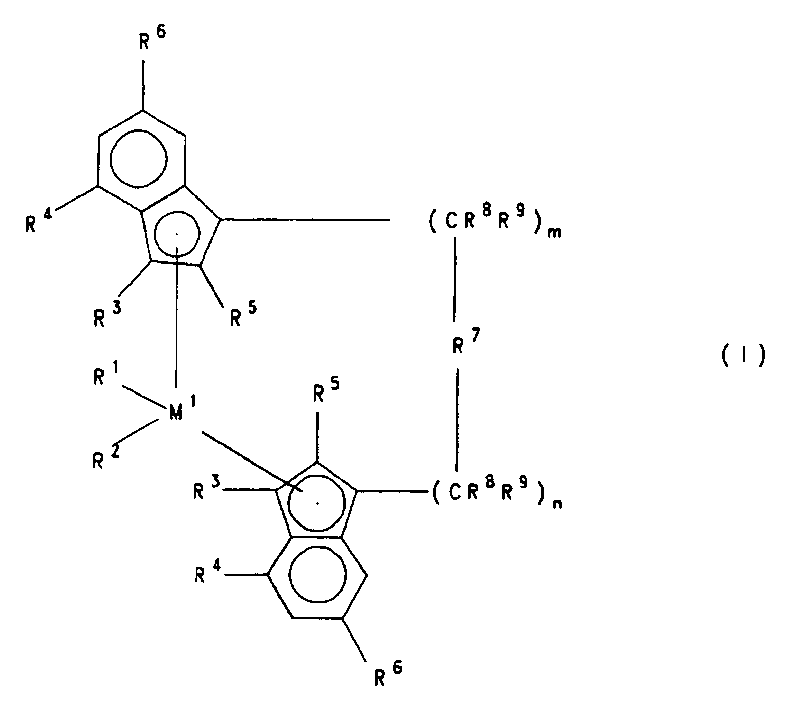 dans laquelle
M¹ représente un métal du groupe IVb, Vb ou VIb de la classification périodique des éléments,
R¹ et R² sont identiques ou différents et représentent un atome d'hydrogène, un groupe alkyle en C₁ à C₁₀, un groupe alcoxy en C₁ à C₁₀, un groupe aryle en C₆ à C₁₀, un groupe aryloxy en C₆ à C₁₀, un groupe alcényle en C₂ à C₁₀, un groupe arylalkyle en C₇ à C₄₀, un groupe alkylaryle en C₇ à C₄₀, un groupe arylalcényle en C₈ à C₄₀, ou un atome d'halogène,
les radicaux R³ représentent un atome d'hydrogène,
R⁴ à R⁶ sont identiques ou différents et représentent un atome d'halogène, un groupe alkyle en C₁ à C₁₀, pouvant être halogéné, un groupe aryle en C₆ à C₁₀, pouvant être halogéné, un radical -NR₂¹⁰, -SR¹⁰, -OSiR₃¹⁰, -SiR₃¹⁰ ou -PR₂¹⁰, où R¹⁰ représente un atome d'halogène, un groupe alkyle en C₁ à C₁₀ ou un groupe aryle en C₆ à C₁₀,
R⁷ représente
R⁷ 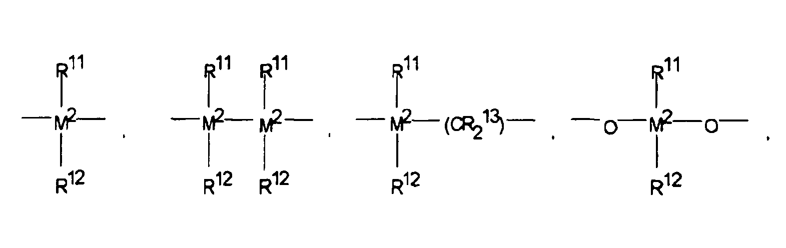 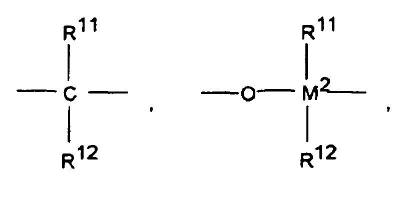 =BR¹¹, =AlR¹¹, -Ge-, -Sn-, -O-, -S-, =SO, =SO₂, =NR¹¹, =CO, =PR¹¹ ou =P(O)R¹¹,
dans lesquelles
R¹¹, R¹² et R¹³ sont identiques ou différents et représentent un atome d'hydrogène, un atome d'halogène, un groupe alkyle en C₁ à C₁₀, un groupe fluoroalkyle en C₁ à C₁₀, un groupe aryle en C₆ à C₁₀, un groupe fluoroaryle en C₆ à C₁₀, un groupe alcoxy en C₁ à C₁₀, un groupe alcényle en C₂ à C₁₀, un groupe arylalkyle en C₇ à C₄₀, un groupe arylalcényle en C₈ à C₄₀, un groupe alkylaryle en C₇ à C₄₀, ou R¹¹ et R¹² ou R¹¹ et R¹³ forment respectivement un cycle avec les atomes auxquels ils sont reliés,
M² représente le silicium, le germanium ou l'étain,
R⁸ et R⁹ sont identiques ou différents et ont la signification indiquée pour R¹¹ et
m et n sont identiques ou différents et représentent zéro, 1 ou 2, la somme m plus n étant égale à zéro, 1 ou 2.

2. Procédé de préparation d'un indène de formule VII ou VIIa 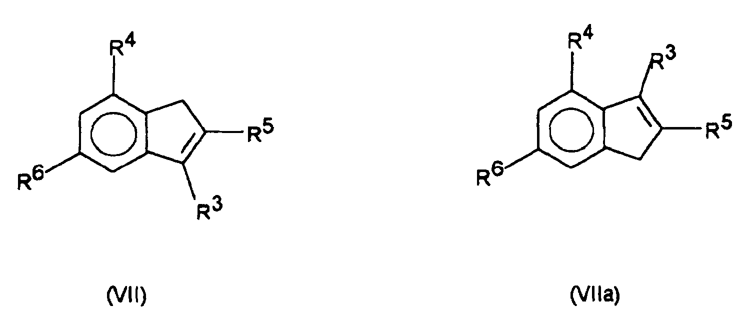 dans laquelle
a) un composé IV 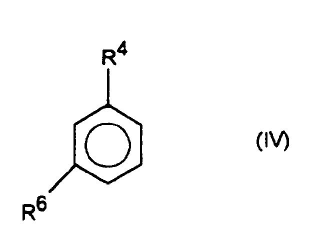 réagit avec un composé V 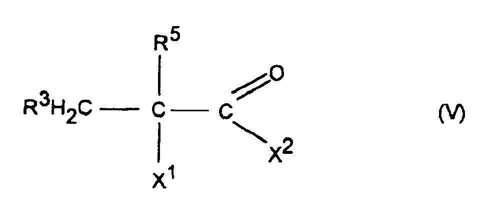 ou son anhydride en présence d'un catalyseur de Friedel - Craft pour donner un indanone VI ou VIa 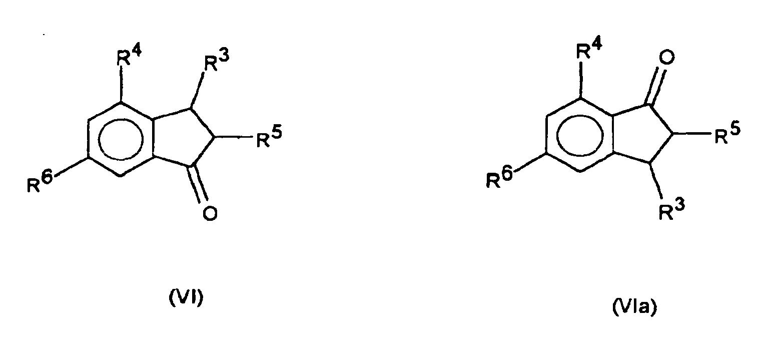 où, dans les formules IV, V, VI, VIa, VII et VIIa, les radicaux R³ à R⁶ sont tels qu'ils sont définis dans la formule I et X¹ et X² représentent un groupe sortant nucléophile, et
b) l'indanone est réduit ensuite avec un agent réducteur pour donner l'indanol correspondant et est ensuite déshydraté.

3. Indène de formule VII ou VIIa 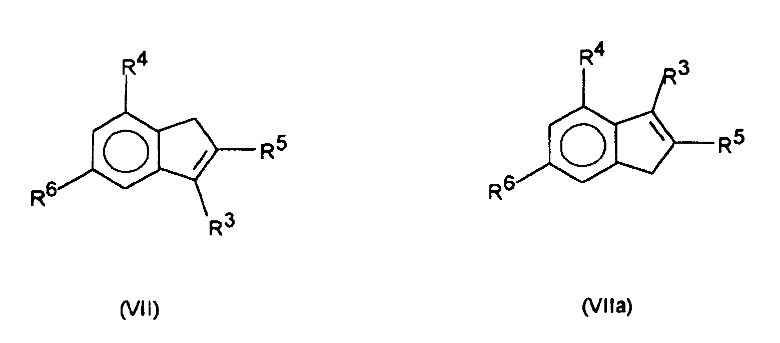 dans laquelle
R³ représente un atome d'hydrogène,
R⁴ à R⁶ sont identiques ou différents et représentent un atome d'halogène, un groupe alkyle en C₁ à C₁₀, pouvant être halogéné, un groupe aryle en C₆ à C₁₀, pouvant être halogéné, un radical -NR₂¹⁰, -SR¹⁰, -OSiR₃¹⁰, -SiR₃¹⁰ ou -PR₂¹⁰, où R¹⁰ représente un atome d'halogène, un groupe alkyle en C₁ à C₁₀ ou un groupe aryle en C₆ à C₁₀, à l'exclusion du 2,4,6-trichloroindène.

4. Indanone de formule VIa 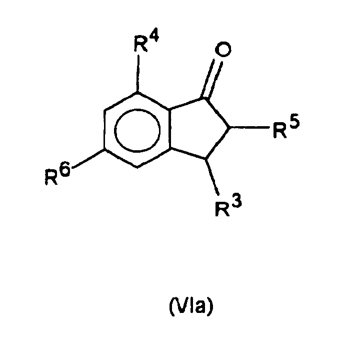 dans laquelle
R³ représente un atome d'hydrogène, un atome d'halogène, un groupe alkyle en C₁ à C₁₀, pouvant être halogéné, un groupe aryle en C₆ à C₁₀, pouvant être halogéné, un radical -NR₂¹⁰, -SR¹⁰, -OSiR₃¹⁰, -SiR₃¹⁰ ou -PR₂¹⁰, où R¹⁰ représente un atome d'halogène, un groupe alkyle en C₁ à C₁₀ ou un groupe aryle en C₆ à C₁₀,
R⁴ à R⁶ sont identiques ou différents et ont la signification citée pour R³, à la condition que R⁴ et R⁶ ne représentent pas un atome d'hydrogène.

5. Indanone selon la revendication 4, dans lequel R³ représente un atome d'hydrogène et R⁵ ne représente pas un atome d'hydrogène.

6. 2-méthyl-4,6-diisopropyl-1-indanone.

7. Utilisation d'un indanone selon la revendication 4 à 6 pour la préparation d'un indène tel qu'il est défini dans la revendication 3.
